(19) 

**Europäisches Patentamt**
**European Patent Office**
**Office européen des brevets**

(11) **EP 4 613 771 A1**

(12) **EUROPEAN PATENT APPLICATION**
published in accordance with Art. 153(4) EPC

(43) Date of publication:
**10.09.2025 Bulletin 2025/37**

(21) Application number: **23886288.2**

(22) Date of filing: **01.11.2023**

(51) International Patent Classification (IPC):
*C07K 16/28* (2006.01)  *C07K 14/725* (2006.01)
*A61P 35/00* (2006.01)  *A61K 39/00* (2006.01)

(52) Cooperative Patent Classification (CPC):
**A61K 39/00; A61P 35/00; C07K 14/705;**
**C07K 16/28**

(86) International application number:
**PCT/KR2023/017306**

(87) International publication number:
**WO 2024/096592 (10.05.2024 Gazette 2024/19)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **02.11.2022   KR 20220144618**

(71) Applicant: **Cellengene Inc**
**Gyeonggi-do 13488 (KR)**

(72) Inventors:
• **AN, Jae Hyung**
  **Seongnam-si, Gyeonggi-do 13488 (KR)**
• **HAN, Na Kyung**
  **Seoul 01913 (KR)**

(74) Representative: **Lavoix**
  **Bayerstraße 83**
  **80335 München (DE)**

Remarks:
The complete document including Reference
Table(s) and the Sequence Listing(s) can be
downloaded from the EPO website

(54) **CHIMERIC ANTIGEN RECEPTOR WITH INCREASED AFFINITY FOR MESOTHELIN AND USE THEREOF**

(57)    Provided is an anti-mesothelin chimeric antigen receptor that has increased affinity for mesothelin and binds specifically to mesothelin. An anti-mesothelin chimeric antigen receptor according to one aspect has increased affinity for mesothelin and exhibits a specific binding ability to mesothelin, and accordingly, can be useful for the prevention or treatment of cancer in which mesothelin is overexpressed.

[FIG 20]

EP 4 613 771 A1

**Description**

Technical Field

The present disclosure relates to an anti-mesothelin chimeric antigen receptor that has increased affinity for mesothelin and binds specifically to mesothelin and use thereof.

Background Art

Recently, immunotherapies such as immune checkpoint inhibitors and CAR-T cell therapies have proven their effectiveness in various cancers. As for solid tumors, however, these new types of immunotherapies do not significantly affect therapy efficiency. This is presumed to be because fibrous tissues surrounding the tumor interferes with the immunotherapy response and makes drug delivery difficult. Therefore, as a specific and more effective CAR-T cancer treatment method, there is a need to develop an antibody that targets a protein specifically overexpressed on the surface of solid cancer cells as a cancer antigen, and has high affinity therefor, and a method of treating cancer using the same to effectively treat solid cancer.

Meanwhile, mesothelin is a glycoprotein anchored on the cell surface by a glycosylphosphatidylinositol (GPI) domain. Normally, mesothelin is expressed at low levels on the mesothelium surrounding the body's cavities and internal organs. However, reportedly, in the case of pancreatic cancer, mesothelioma, ovarian cancer, and non-small cell lung cancer, mesothelin is expressed at high levels.

Detailed Description of the present disclosure

Technical Problem

One aspect is to provide an anti-mesothelin antibody or an antigen-binding fragment thereof, with increased affinity for mesothelin.

Another aspect is to provide an isolated nucleic acid encoding the anti-mesothelin antibody or the antigen-binding fragment thereof, with increased affinity for mesothelin.

Another aspect is to provide a vector including the isolated nucleic acid.

Another aspect is to provide isolated host cells transformed with the vector.

Another aspect is to provide a method of producing an anti-mesothelin antibody with increased affinity for mesothelin, including culturing the isolated host cells to express an antibody.

Another aspect is to provide a chimeric antigen receptor with increased affinity for mesothelin, including an antigen-binding domain, a hinge domain, a transmembrane domain, and an intracellular signaling domain.

Another aspect is to provide a polynucleotide encoding the chimeric antigen receptor.

Another aspect is to provide a vector including the polynucleotide.

Another aspect is to provide isolated cells transformed with the vector.

Another aspect is to provide: a pharmaceutical composition including the isolated cells, medicinal use of the cells, and a method of preventing or treating cancer, including administering to a subject a therapeutically effective amount of the cells.

Other objects and advantages of the present application will become clearer from the following detailed description together with the appended claims and drawings. Contents not described in this specification could be fully recognized and inferred by a person skilled in the technical field of this application or a technical field that is similar thereto. Accordingly, the relevant description will be omitted.

Technical Solution to Problem

Each description and embodiment disclosed in this application can also be applied to other descriptions and embodiments. That is, all combinations of the various elements disclosed in this application fall within the scope of this application. Additionally, the scope of the present application may not be considered limited by the specific description described below.

One aspect provides an anti-mesothelin antibody or an antigen-binding fragment thereof, including: a heavy chain variable region including heavy chain complementarity determining region 1 (HCDR1) including an amino acid sequence consisting of SEQ ID NO: 19, heavy chain complementarity determining region 2 (HCDR2) including an amino acid sequence consisting of SEQ ID NO: 20, and a heavy chain complementarity determining region 3 (HCDR3) including an amino acid sequence consisting of SEQ ID NO: 21; and a light chain variable region including light chain complementarity determining region 1 (LCDR1) including an amino acid sequence consisting of SEQ ID NO: 22, light chain complementarity determining region 2 (LCDR2) including an amino acid sequence consisting of SEQ ID NO: 23, and light chain complementarity determining region 3 (LCDR3) including an amino acid sequence consisting of SEQ ID NO: 24, wherein the heavy chain variable region and the light chain variable region include one or more amino acid substitutions.

The "mesothelin (MSLN)" is a cell surface glycoprotein with a total amino acid length of 622aa (NCBI Gene ID: 10232), and is selectively expressed in some cells, especially certain tumor cells. The amino acids of mesothelin protein are as follows:

MALPTARPLLGSCGTPALGSLLFLLFSLGWVQPSRTLAGETGQEAAPLDGVLA
NPPNISSLSPRQLLGFPCAEVSGLSTERVRELAVALAQKNVKLSTEQLRCLAHRLSEPP
EDLDALPLDLLLFLNPDAFSGPQACTRFFSRITKANVDLLPRGAPERQRLLPAALACWG
VRGSLLSEADVRALGGLACDLPGRFVAESAEVLLPRLVSCPGPLDQDQQEAARAALQ
GGGPPYGPPSTWSVSTMDALRGLLPVLGQPIIRSIPQGIVAAWRQRSSRDPSWRQPE
RTILRPRFRREVEKTACPSGKKAREIDESLIFYKKWELEACVDAALLATQMDRVNAIPFT
YEQLDVLKHKLDELYPQGYPESVIQHLGYLFLKMSPEDIRKWNVTSLETLKALLEVNKG
HEMSPQVATLIDRFVKGRGQLDKDTLDTLTAFYPGYLCSLSPEELSSVPPSSIWAVRP
QDLDTCDPRQLDVLYPKARLAFQNMNGSEYFVKIQSFLGGAPTEDLKALSQQNVSMD
LATFMKLRTDAVLPLTVAEVQKLLGPHVEGLKAEERHRPVRDWILRQRQDDLDTLGLG
LQGGIPNGYLVLDLSMQEALSGTPCLLGPGPVLTVLALLLASTLA (SEQ ID NO: 60)

Mesothelin is expressed at low levels in normal mesothelial cells, but is highly expressed in solid cancer (solid tumors), and the high expression thereof in esophageal cancer, breast cancer, triple-negative breast cancer (TNBC), gastric cancer, cholangiocarcinoma, pancreatic cancer, colon cancer, lung cancer, thymic carcinoma, mesothelioma, ovarian cancer, endometrial cancer, cervical cancer, uterine serous carcinoma (USC), and pediatric acute myeloid leukemia (AML) was confirmed (Cancer Discov. 2016 Feb; 6(2):133-46.;J Reprod Immunol. 2020;139:103115.; Gynecol Oncol. 2007;105(3):563-570.; Eur J Haematol. 2007;79(4):281-286.).

The term "antibody" as used herein refers to a general term for proteins that selectively act on antigens and participate in biological immunity, and types thereof are not particularly limited. The heavy chain and the light chain of antibodies have antigen-binding sites that recognize epitopes and include a variable region, and antigen specificity appears according to changes in the sequence of the variable region. The variable region of the antigen-binding site is divided into a less variable framework region (FR) and a more variable complementarity determining region (CDR), and both the heavy chain and the light chain each include three CDR regions, that is, CDR1, CDR2, and CDR3, and four FR regions. The CDRs of each chain are typically named CDR1, CDR2, and CDR3 sequentially starting from the N-terminus, and are also identified by the chain on which a specific CDR is located.

The term "complementarity determining region" as used herein refers to a region in the variable region of an antibody that confers binding specificity to an antigen.

The term "epitope" as used herein refers to a specific three-dimensional molecular structure within an antigen molecule to which an antibody can specifically bind.

The antibodies include monoclonal antibodies, multispecific antibodies, human antibodies, humanized antibodies, and chimeric antibodies (for example, humanized murine antibodies). Additionally, the antibodies may include diabodies, triabodies, and tetrabodies.

The term "an antibody" as used herein includes an "antigen-binding fragment" or "antibody fragment" of an antibody that possesses antigen-binding ability. The antigen-binding fragment may be an antibody fragment including one or more complementarity determining region, and such an antibody fragment may be selected from the group consisting of scFv, (scFv)2, scFv-Fc, Fab, Fab', and F(ab')2. Regarding an antibody fragment, Fab includes the variable regions of the light and heavy chains, the constant region of the light chain, and the first constant region (CH1) of the heavy chain, and has one antigen-binding site. Fab' differs from Fab in that a hinge region including one or more cysteine residues is located at the C-terminus of the heavy chain CH1 domain. F(ab')2 antibody is produced when cysteine residues in the hinge region of Fab' form a disulfide bond. Fv is the smallest antibody fragment including only a heavy chain variable region and a light chain variable region. Two-chain Fv consists of a heavy chain variable region and a light chain variable region connected by a non-covalent bond. Single-chain Fv (scFv) generally may have a dimer-like structure like two-chain Fv in which the variable region of the heavy chain and the variable region of the light chain are covalently linked through a peptide linker or are directly linked at the C-terminus.

In an embodiment, the anti-mesothelin antibody or the antigen-binding fragment thereof includes one or more amino acid substitutions, wherein the one or more amino acid substitutions may include about 1 to about 5 amino acids, about 1 to about 4 amino acids, about 1 to about 3 amino acid substitutions, about 1 to about 2 amino acid substitutions, in an amino acid of SEQ ID NO: 1.

The one or more amino acid substitutions may occur in one or more positions selected from the group consisting of a 1st position of SEQ ID NO: 19 (1st amino acid of HCDR1), a 7th position of SEQ ID NO: 23 (7th amino acid of LCDR2), and a 4th position of SEQ ID NO: 24 (4th amino acid of LCDR3).

In an embodiment, the one or more amino acid substitutions may include one or more selected from the group consisting of 1) to 3) below:

1) the 1st amino acid of SEQ ID NO: 19 is substituted from D to K, W, L or R,
2) the 7th amino acid of SEQ ID NO: 23 is substituted from S to F or R; and
3) the 4th amino acid of SEQ ID NO: 24 is substituted from Y to R.

In the present specification, the 1st amino acid of SEQ ID NO: 19 may be substituted from D to K, W, L, or R, which are expressed as D31L, D31K, D31W, or D31R, respectively, and the 7th amino acid of SEQ ID NO: 23 may be substituted from S to F or R, which are expressed as S192F or S192R, respectively, and the 4th amino acid of SEQ ID NO: 24 may be substituted from Y to R, which is expressed as Y228R.

In an embodiment, the one or more amino acid substitutions may be one or more selected from the group consisting of 1) to 3) below:

1) the 1st amino acid of SEQ ID NO: 19 is substituted from D to L,
2) the 7th amino acid of SEQ ID NO: 23 is substituted from S to R; and
3) the 1st amino acid of SEQ ID NO: 19 is substituted from D to L, and the 7th amino acid of SEQ ID NO: 23 is substituted from S to R.

In an embodiment, the anti-mesothelin antibody or the antigen-binding fragment thereof may be selected from antibodies or antigen-binding fragments thereof, including a heavy chain variable region including heavy chain CDRs and a light chain variable region including light chain CDRs:

1) an antibody or an antigen-binding fragment thereof, including: a heavy chain variable region including HCDR1 including the amino acid sequence consisting of SEQ ID NO: 27, HCDR2 including the amino acid sequence consisting of SEQ ID NO: 28, and HCDR3 including the amino acid sequence consisting of SEQ ID NO: 29; and a light chain variable region including LCDR1 including the amino acid sequence consisting of SEQ ID NO: 30, LCDR2 including the amino acid sequence consisting of SEQ ID NO: 31, and LCDR3 including the amino acid sequence consisting of SEQ ID NO: 32,
2) an antibody or an antigen-binding fragment thereof, including: a heavy chain variable region including HCDR1 including the amino acid sequence consisting of SEQ ID NO: 35, HCDR2 including the amino acid sequence consisting of SEQ ID NO: 36, and HCDR3 including the amino acid sequence consisting of SEQ ID NO: 37; and a light chain variable region including LCDR1 including the amino acid sequence consisting of SEQ ID NO: 38, LCDR2 including the amino acid sequence consisting of SEQ ID NO: 39, and LCDR3 including the amino acid sequence consisting of SEQ ID NO: 40, and
3) an antibody or an antigen-binding fragment thereof, including: a heavy chain variable region including HCDR1 including the amino acid sequence consisting of SEQ ID NO: 43, HCDR2 including the amino acid sequence consisting of SEQ ID NO: 44, and HCDR3 including the amino acid sequence consisting of SEQ ID NO: 45; and a light chain variable region including LCDR1 including the amino acid sequence consisting of SEQ ID NO: 46, LCDR2 including the amino acid sequence consisting of SEQ ID NO: 47, and LCDR3 including the amino acid sequence consisting of SEQ ID NO: 48.

In an embodiment, the anti-mesothelin antibody or the antigen-binding fragment thereof may be selected from antibodies or antigen-binding fragments thereof including the following heavy chain variable region and the following light chain variable region:

1) an antibody or an antigen-binding fragment thereof including a heavy chain variable region including the amino acid sequence consisting of SEQ ID NO: 33 and a light chain variable region including the amino acid sequence consisting of SEQ ID NO: 34,

2) an antibody or an antigen-binding fragment thereof including a heavy chain variable region including the amino acid sequence consisting of SEQ ID NO: 41 and a light chain variable region including the amino acid sequence consisting of SEQ ID NO: 42, and

3) an antibody or an antigen-binding fragment thereof including a heavy chain variable region including the amino acid sequence consisting of SEQ ID NO: 49 and a light chain variable region including the amino acid sequence consisting of SEQ ID NO: 50.

In an embodiment, the anti-mesothelin antibody or the antigen-binding fragment thereof may be or include a single chain variable fragment (scFv), and the anti-mesothelin scFv including one or more amino acid substitutions may include a substitution selected from the group consisting of the following amino acid substitutions:

1) the 31st amino acid of SEQ ID NO: 1 is substituted from D to L (D31L),
2) the 192nd amino acid of SEQ ID NO: 1 is substituted from S to R (S192R), and
3) the 31st amino acid in SEQ ID NO: 1 is substituted from D to L, and the 192nd amino acid in SEQ ID NO: 1 is substituted from S to R (D31L/S192R).

In an embodiment, the anti-mesothelin antibody or the antigen-binding fragment thereof may be selected from antibodies or antigen-binding fragments thereof including the following antigen-binding fragments:

1) an antibody or an antigen-binding fragment thereof, including an antigen-binding fragment that includes the amino acid sequence consisting of SEQ ID NO: 2,
2) an antibody or antigen-binding fragment thereof, including an antigen-binding fragment that includes the amino acid sequence consisting of SEQ ID NO: 3, and
3) an antibody or an antigen-binding fragment thereof, including an antigen-binding fragment that includes the amino acid sequence consisting of SEQ ID NO: 4.

Table 1

| Type | Amino acid sequence | SEQ ID NO. |
|---|---|---|
| MSLN34 | EVQLVESGGGLVQPGGSLRLSCAASGFTFSDYGMHWVRQAPGKGLEWVSSIYGSGGHTGYADSVKGRFTISRDNSKNTLYLQMNSLRAEDTAVYYCAKQHAYRYSYAFDVWGQGTLVTVSSGGGGSGGGGSGGGGSDIQMTQSPSSLSASVGDRVTITCRASQSISNWLNWYQQKPGKAPKLLIYATSSLQSGVPSRFSGSGSGTDFTLTISSLQPEDFATYYCQQSYSFPFTFGQGTKVEIK | 1 |
| MSLN34 D31L | EVQLVESGGGLVQPGGSLRLSCAASGFTFSLYGMHWVRQAPGKGLEWVSSIYGSGGHTGYADSVKGRFTISRDNSKNTLYLQMNSLRAEDTAVYYCAKQHAYRYSYAFDVWGQGTLVTVSSGGGGSGGGGSGGGGSDIQMTQSPSSLSASVGDRVTITCRASQSISNWLNWYQQKPGKAPKLLIYATSSLQSGVPSRFSGSGSGTDFTLTISSLQPEDFATYYCQQSYSFPFTFGQGTKVEIK | 2 |

(continued)

| Type | Amino acid sequence | SEQ ID NO. |
|---|---|---|
| MSLN34 S192R | EVQLVESGGGLVQPGGSLRLSCAASGFTFSDYGMH WVRQAPGKGLEWVSSIYGSGGHTGYADSVKGRFTI SRDNSKNTLYLQMNSLRAEDTAVYYCAKQHAYRYS YAFDVWGQGTLVTVSSGGGGSGGGGSGGGGSDIQ MTQSPSSLSASVGDRVTITCRASQSISNWLNWYQQ KPGKAPKLLIYATSSLQRGVPSRFSGSGSGTDFTLTI SSLQPEDFATYYCQQSYSFPFTFGQGTKVEIK | 3 |
| MSLN34 D31L/S192R | EVQLVESGGGLVQPGGSLRLSCAASGFTFSLYGMH WVRQAPGKGLEWVSSIYGSGGHTGYADSVKGRFTI SRDNSKNTLYLQMNSLRAEDTAVYYCAKQHAYRYS YAFDVWGQGTLVTVSSGGGGSGGGGSGGGGSDIQ MTQSPSSLSASVGDRVTITCRASQSISNWLNWYQQ KPGKAPKLLIYATSSLQRGVPSRFSGSGSGTDFTLTI SSLQPEDFATYYCQQSYSFPFTFGQGTKVEIK | 4 |

The anti-mesothelin antibody or the antigen-binding fragment thereof may include a heavy chain variable region having, with the amino acid sequence consisting of SEQ ID NOS: 33, 41 or 49, at least 80% sequence homology, at least 90% sequence homology, at least 95% sequence homology, or 100% sequence homology.

The anti-mesothelin antibody or the antigen-binding fragment thereof may include a light chain variable region having, with the amino acid sequence consisting of SEQ ID NOS: 34, 42, or 50, at least 80% sequence homology, at least 90% sequence homology, at least 95% sequence homology, or 100% sequence homology.

Considering mutations having biologically equivalent activity, an antibody or nucleic acid molecule encoding the same may be interpreted as including a sequence showing substantial identity with the sequence shown in the sequence number. The substantial identity may refer to at least 61% homology, 70% homology, 80% homology, 90% homology, 95% homology, 98% homology, or 99% homology, when the sequence and any other sequence are aligned to correspond to each other as much as possible, and the aligned sequence is analyzed using an algorithm commonly used in the art. Alignment methods for sequence comparison are known in the art.

When one or more amino acid substitutions are included, the affinity for mesothelin may be increased, resulting in improved mesothelin affinity. When one or more amino acid substitutions are included, the binding affinity to the target mesothelin may be increased compared to the wild type that does not include such substitutions.

Another aspect provides an anti-mesothelin antibody or an antigen-binding fragment thereof, including one or more amino acid substitutions in the amino acid sequence consisting of SEQ ID NO: 1. The same description above is equally applied to the antibody or the antigen-binding fragment thereof.

In an embodiment, the anti-mesothelin antibody or the antigen-binding fragment thereof may be an anti-mesothelin scFv including one or more amino acid substitutions in the amino acid sequence consisting of SEQ ID NO: 1. The one or more amino acid substitutions may include about 1 to about 5, about 1 to about 4, about 1 to about 3, or about 1 to about 2 amino acid substitutions within the amino acid of SEQ ID NO: 1.

The one or more amino acid substitutions may occur at one or more positions selected from the group consisting of the 31st position, the 192nd position, and the 228th position of SEQ ID NO: 1. For example, the one or more amino acid substitutions of the anti-mesothelin antibody or antigen-binding fragment thereof may be one or more selected from the group consisting of the following groups (group a to group c):

a) D31K, D31W, D31L and D31R;

b) S192F and S192R; and
c) Y228R.

When one or more amino acid substitutions are included, the affinity for mesothelin may be increased, resulting in improved mesothelin affinity. When one or more amino acid substitutions are included, the binding affinity to the target mesothelin may be increased compared to the wild type that does not include such substitutions.

The anti-mesothelin scFv including one or more amino acid substitutions may be, for example, one or more selected from the group consisting of SEQ ID NOS: 2, 3, and 4.

Another aspect provides isolated nucleic acid encoding the antibody or the antigen-binding fragment thereof. The same description above is equally applied to the nucleic acid.

The term "nucleic acid" as used herein comprehensively includes DNA and RNA molecules, and nucleotides, which are the basic structural units in nucleic acids, include not only natural nucleotides but also analogues in which sugar or base sites have been modified. The sequences of a nucleic acid encoding a heavy variable region and a light chain variable region of one aspect may be modified. The modifications may include additions, deletions, or non-conservative, or conservative substitutions of nucleotides.

The nucleic acid may be interpreted to also include a nucleotide sequence showing substantial identity to the nucleotide sequence of the nucleic acid. Substantial identity indicates a nucleotide sequence showing at least 80% homology, at least 90% homology, and 95% homology, when a nucleotide sequence of one aspect and any other sequence are aligned to correspond to each other as much as possible, and the aligned sequence is analyzed using an algorithm commonly used in the art.

Another aspect provides a vector including the isolated nucleic acid. The same description above is equally applied to the vector.

For the expression of an antibody or an antibody fragment thereof in a suitable host cell, the vector may be prepared from DNA encoding partial or full-length light and heavy chains using standard molecular biology techniques (e.g., PCR amplification or cDNA cloning using hybridoma expressing a target antibody), and the vector may include a necessary regulatory element operably linked such that DNA (gene) insert can be expressed. "Operably linked" refer to a functional linkage between a nucleic acid expression control sequence and a nucleic acid sequence encoding a target protein or RNA in which the linkage is made such that a gene is able to be expressed by an expression control sequence.

The "expression control sequence" refers to a DNA sequence that regulates the expression of an operably linked DNA sequence in a specific host cell. Such control sequences include a promoter to effect transcription, an optional operator sequence to regulate transcription, a sequence encoding a suitable mRNA ribosome binding site, a sequence regulating termination of transcription and translation, an initiation codon, a stop codon, a polyadenylation signal, and an enhancer. Those skilled in the art will recognize that the design of the expression vector may vary by selecting different control sequences depending on factors such as the selection of the host cell to be transformed, the expression level of the protein, etc.

The type of the vector is not particularly limited as long as it is a vector commonly used in the cloning and antibody production fields, and examples thereof are, but are not limited to, plasmid vectors, cosmid vectors, bacteriophage vectors, and viral vectors. The plasmids may be E. coli-derived plasmids (pBR322, pBR325, pUC118, and pUC119, pET-21b(+)), Bacillus subtilis-derived plasmids (pUB110 and pTP5), and yeast-derived plasmids (YEp13, YEp24, and YCp50). The virus may be an animal virus such as a retrovirus, an adenovirus, or a vaccinia virus, or an insect virus such as a baculovirus. pComb3 series vector commonly used for phage display, etc. may be used, and to express antibodies in mammalian cells, vectors commonly used to express proteins in mammalian cells, such as pcDNA or pVITRO, may be used.

Another aspect provides an isolated host cell transformed with the vector. The same description above is equally applied to the host cell.

The term "transformation" as used herein refers to molecular biological technology in which a DNA chain fragment or plasmid including a foreign gene of a different type from that of the original cell is infiltrated between cells to combine with the DNA present in the original cell so as to change the genetic inheritance of a cell. The vector is transfected into a host cell. For the transfection, many different techniques commonly used to introduce exogenous nucleic acids (DNA or RNA) into prokaryotic or eukaryotic host cells may be used. Such techniques are electrophoresis, calcium phosphate precipitation, and DEAE-dextran transfection or lipofection.

The antibody or antigen-binding fragment thereof according to one aspect may be expressed in eukaryotic cells, or mammalian host cells, considering applicability to microorganisms such as bacteria (*E. coli*) or yeast, or mammalian cells. The mammalian host cell may be, for example, one selected from the group consisting of Chinese hamster ovary (CHO) cells, NSO myeloma cells, COS cells, SP2 cells, F2N cells, HEK293 cells, and antibody-producing hybridoma cells, and is not limited thereto.

Another aspect is to provide a method of producing an anti-mesothelin antibody with increased affinity for mesothelin, including culturing the isolated host cells to express an antibody. The same description above is equally applied to the method.

The method may include transforming a host cell for producing an antibody or antigen-binding fragment thereof according to an aspect with a vector in which DNA encoding the antibody or the antigen-binding fragment is operably linked. The types of host cells and recombinant expression vectors selected are as described above, and this process will be performed by selecting an appropriate transformation method. In the case where a recombinant expression vector encoding the antibody gene is introduced into a mammalian host cell, an antibody may be produced by culturing a host cell during a period sufficient to cause the antibody to be expressed in the host cell or during a period sufficient to cause the antibody to be secreted into the culture medium in which the host cell is cultured.

In addition, the method may additionally include culturing the transformed, isolated host cell to produce a polypeptide of the antibody or antigen-binding fragment thereof according to one aspect from the recombinant expression vector introduced into the host cell. The medium composition, culture conditions, and culture time for culturing the selected host cells may be appropriately selected, and antibody molecules produced in the host cells may accumulate in a cell cytoplasm, or may be secreted outside the cell or into the culture medium by an appropriate signal sequence, or may be targeted with periplasm, etc. In some embodiments, in order for the antibody according to one aspect to maintain binding specificity for mesothelin, the protein refolding may be cause to occur using methods known in the art to have a functional structure. In some embodiments, in the case where an antibody in the form of IgG is formed, a heavy chain and a light chain may be expressed in separate cells and brought into contact with each other in a separate step to form a complete antibody, or a heavy chain and a light chain may be expressed in the same cell to form a complete antibody inside the cell.

In some embodiments, the method may additionally include obtaining an antibody or an antigen-binding fragment thereof produced in an isolated host cell. The obtaining method may be appropriately selected and adjusted by considering the characteristics of the polypeptide of the antibody or antigen-binding fragment thereof produced in the host cell, the characteristics of the host cell, the expression method, or whether the polypeptide is targeted. For example, antibodies or antigen-binding fragments thereof secreted into the culture medium may be recovered by obtaining a medium in which host cells are cultured, and centrifuging to remove impurities, and, if necessary, to release and recover antibodies present in specific organelles or cytoplasm outside the cells, cells may be lysed to the extent that the functional structure of an antibody or an antigen-binding fragment thereof is not affected.

The obtained antibody may be further subjected to a process of further removing impurities and concentrating through, for example, filtration using chromatography or filters, etc., and dialysis. Separation or purification of the obtained antibody may be performed by conventional separation and purification methods used for proteins, for example, chromatography. The chromatography may include, for example, affinity chromatography, ion exchange chromatography, or hydrophobic chromatography, including a Protein A column, Protein G column, and a Protein L column. In addition to the chromatography, antibodies may be separated and purified by additionally combining filtration, ultrafiltration, salting out, dialysis, etc.

Another aspect provides a chimeric antigen receptor including an antigen-binding domain, a hinge domain, a transmembrane domain, and an intracellular signaling domain, wherein the antigen-binding domain includes the anti-mesothelin antibody or the antigen-binding fragment thereof. The same description above is equally applied to the chimeric antigen receptor. Since the chimeric antigen receptor binds specifically to mesothelin, the chimeric antigen receptor may include an antigen-binding domain that binds specifically to mesothelin.

An antigen-binding domain may include an anti-mesothelin antibody or an antigen-binding fragment thereof, including: a heavy chain variable region including heavy chain complementarity determining region 1 (HCDR1) including an amino acid sequence consisting of SEQ ID NO: 19, heavy chain complementarity determining region 2 (HCDR2) including an amino acid sequence consisting of SEQ ID NO: 20, and a heavy chain complementarity determining region 3 (HCDR3) including an amino acid sequence consisting of SEQ ID NO: 21; and a light chain variable region including light chain complementarity determining region 1 (LCDR1) including an amino acid sequence consisting of SEQ ID NO: 22, light chain complementarity determining region 2 (LCDR2) including an amino acid sequence consisting of SEQ ID NO: 23, and light chain complementarity determining region 3 (LCDR3) including an amino acid sequence consisting of SEQ ID NO: 24, wherein the heavy chain variable region and the light chain variable region include one or more amino acid substitutions.

In an embodiment, the one or more amino acid substitutions may include one or more selected from the group consisting of 1) to 3) below:

    1) the 1st amino acid of SEQ ID NO: 19 is substituted from D to K, W, L or R;
    2) the 7th amino acid of SEQ ID NO: 23 is substituted from S to F or R; and
    3) the 4th amino acid of SEQ ID NO: 24 is substituted from Y to R.

In the present specification, the 1st amino acid of SEQ ID NO: 19 may be substituted from D to K, W, L, or R, which are expressed as D31L, D31K, D31W, or D31R, respectively, and the 7th amino acid of SEQ ID NO: 23 may be substituted from S to F or R, which are expressed as S192F or S192R, respectively, and the 4th amino acid of SEQ ID NO: 24 may be substituted from Y to R, which is expressed as Y228R.

In an embodiment, the one or more amino acid substitutions may be one or more selected from the group consisting of 1) to 3) below:

1) the 1st amino acid of SEQ ID NO: 19 is substituted from D to L;
2) the 7th amino acid of SEQ ID NO: 23 is substituted from S to R; and
3) the 1st amino acid of SEQ ID NO: 19 is substituted from D to L, and the 7th amino acid of SEQ ID NO: 23 is substituted from S to R.

When one or more amino acid substitutions are included, the affinity for mesothelin may be increased, resulting in improved mesothelin affinity. When one or more amino acid substitutions are included, the binding affinity to the target mesothelin may be increased compared to the wild type that does not include such substitutions.

When one or more amino acid substitutions are included, the affinity for mesothelin may be increased, resulting in improved mesothelin affinity. When one or more amino acid substitutions are included, the binding affinity to the target mesothelin may be increased compared to the wild type that does not include such substitutions. In addition, in the case where one or more amino acid substitutions are included, excellent cytotoxic ability against cancer cells expressing mesothelin, such as mesothelioma, ovarian cancer, and pancreatic cancer, may occur.

The term "chimeric antigen receptor (CAR)" as used herein refers to such a structure that an antigen-binding (recognition) domain, a transmembrane domain, and an intracellular signaling domain are included to constitute a chimeric antigen receptor.

In an embodiment, the antigen-binding fragment may be a single chain variable fragment (scFv).

In an embodiment, the antigen-binding domain may be selected from antibodies including the following antigen-binding fragment or antigen-binding fragments thereof:

1) an antibody or an antigen-binding fragment thereof, including an antigen-binding fragment that includes the amino acid sequence consisting of SEQ ID NO: 2;
2) an antibody or antigen-binding fragment thereof, including an antigen-binding fragment that includes the amino acid sequence consisting of SEQ ID NO: 3; and
3) an antibody or an antigen-binding fragment thereof, including an antigen-binding fragment that includes the amino acid sequence consisting of SEQ ID NO: 4.

The hinge domain, transmembrane domain, and intracellular signaling domain included in the chimeric antigen receptor are well known in the art.

The hinge domain is a domain that connects an anti-mesothelin antibody or an antigen-binding fragment thereof with a transmembrane domain, and is also called a spacer, and has the purpose of expanding the antigen-binding domain from a T cell membrane. The hinge domain may be a CD8 hinge domain, an IgG1 hinge domain, an IgG4 hinge domain, a CD28 extracellular region, a killer immunoglobulin-like receptor (KIR) extracellular region, or a combination thereof, and is not limited thereto. The hinge domain may be any hinge domain that is commonly used in the art.

The transmembrane domain acts as a support for the chimeric antigen receptor molecule and simultaneously may link the hinge domain to the intracellular signaling domain. The transmembrane domain may penetrate a cell membrane of a cell such that the anti-mesothelin antibody or the antigen-binding fragment thereof of the chimeric antigen receptor is located on the cell surface and the intracellular signaling domain is located within the cell. The transmembrane domain may be the transmembrane region of CD3 zeta (CD3z), CD4, CD8, CD28 or KIR protein, or the transmembrane domain of CD8 or CD28. However, any typical transmembrane domain that is used in the production of chimeric antigen receptors may be used herein without restrictions.

The intracellular signaling domain may receive signals transmitted by anti-mesothelin antibodies or antigen-binding fragments thereof and deliver the same into cells to which chimeric antigen receptors are bound. The intracellular signaling domain is not particularly limited in type as long as it is a part that transmits a signal that can lead to T cell activation when an antibody binds to an antigen-binding site existing outside the cell. Various types of intracellular signaling domains may be used herein. The intracellular signaling domain may be, for example, an immunoreceptor tyrosine-based activation motif or ITAM, and the ITAM may be derived from CD3 zeta, FcR gamma, FcR beta, CD3 gamma, CD3 delta, and CD3 epsilon, CDS, CD22, CD79a, CD79b, CD66d, or FcεRIγ, and is not limited thereto.

In some embodiments, the chimeric antigen receptor according to one aspect may additionally include a costimulatory domain in addition to the intracellular signaling domain.

The costimulatory domain is a part that transmits signals to T cells in addition to signals by the intracellular signaling domain, and is the intracellular part of the chimeric antigen receptor, including the intracellular domain of the costimulatory molecule.

The costimulatory molecule is a cell surface molecule and refers to a molecule necessary to bring about a sufficient response of lymphocytes to an antigen, and may be, for example, CD27, CD28, 4-1BB, OX40, CD30, CD40, PD-1, ICOS, lymphocyte function-associated antigen-1 (LFA- 1), CD2, CD7, LIGHT, NKG2C, or B7-H3, and is not limited thereto. The costimulatory domain may be an intracellular part of a molecule selected from the group consisting of such costimulatory molecules and combinations thereof.

Each domain of the chimeric antigen receptor, including the transmembrane domain and intracellular signaling domain, may be optionally linked by a short oligopeptide or polypeptide linker. The linker may be any linker that is not particularly limited in length and is known in the art, as long as being able to induce T cell activation through an intracellular domain when the antigen located outside the cell binds to an antibody.

Additionally, the chimeric antigen receptor may include modified forms of the antibodies and domains as described above. In this regard, the modification may be performed by substituting, deleting, or adding one or more amino acids in the amino acid sequence of the wild-type antibody and domain without modifying the function of the antibodies and domains. Typically, the substitution may be alanine or may be performed by conservative amino acid substitution that does not affect the charge, polarity or hydrophobicity of the entire protein.

Another aspect provides a polynucleotide encoding the chimeric antigen receptor. The same description above is equally applied to the polynucleotide.

As for the polynucleotide, due to codon degeneracy or in consideration of codons preferred in organisms intended to express an antigen receptor, a coding region may undergo various modifications within such a range that the amino acid sequence of the antigen receptor expressed from the coding region is not changed, and even parts other than the coding region may undergo various change or modifications within such a range that the expression of the gene is not affected, and such modified genes are also included within the scope of the present disclosure. All of these may be understood well by those skilled in the art. That is, the polynucleotide according to one aspect may be mutated by substitution, deletion, or insertion of one or more nucleic acid bases, or a combination thereof, as long as the polynucleotide encodes a protein with equivalent activity, which may also be included within the scope of the present disclosure.

Another aspect provides a vector including the polynucleotide and an isolated cell transformed with the vector. The same description above is equally applied to the cells.

The vector may be selected from various vectors known in the art, and depending on the type of host cell to be used to produce the antigen receptor, an expression control sequence such as a promoter, terminator, enhancer, etc. or sequences for membrane targeting or secretion may be appropriately selected and combined in various ways depending on the purpose. Vectors of the present disclosure include, but are not limited to, plasmid vectors, cosmid vectors, bacteriophage vectors, viral vectors, etc. In addition to expression control elements such as promoters, operators, start codons, stop codons, polyadenylation signals, and enhancers, suitable vectors may further include signal sequences or leader sequences for membrane targeting or secretion, and can be prepared in various ways depending on the purpose.

In addition, the vector can be introduced into the cell to transform the cell, and the isolated cell may be, but is not limited to, T cells, NK cells, NKT cells, or gamma delta ($\gamma\delta$) T cells. The isolated cells may be obtained or prepared from bone marrow, peripheral blood, peripheral blood mononuclear cells, or umbilical cord blood.

Another aspect is to provide: a pharmaceutical composition including the isolated cells, medicinal use of the isolated cells, and a method of preventing or treating cancer, including administering to a subject a therapeutically effective amount of the isolated cells. The same description above is equally applied to the composition and the method.

Since the pharmaceutical composition uses the isolated cells described above, the common description of these two will be omitted to avoid excessive complexity of the specification.

The pharmaceutical composition or medicinal use may be for the prevention or treatment of cancer.

The term "prevention" as used herein refers to all actions that suppress or delay the onset of cancer (tumor) by administering the pharmaceutical composition according to the present disclosure.

The term "treatment" as used herein refers to all actions that alleviate or beneficially change symptoms with respect to cancer (tumor) by administration of the pharmaceutical composition according to the present disclosure.

The term "subject" as used herein refers to a subject in need of treatment for a disease, and more specifically, a human or non-human primate, or mammals such as rodent (rat, mouse, guinea pig, etc.), mouse, dog, cats, horses, cows, sheep, pigs, goats, camels, and antelopes.

The term "cancer" as used herein refers to a general term for diseases caused by cells with aggressive characteristics in which cells divide and grow ignoring normal growth limits, invasive characteristics in which cells infiltrate surrounding tissues, and metastatic characteristics in which cells spread to other parts of the body. The term "cancer" as used herein is used in the same sense as malignant tumor, and may be mesothelin-positive or mesothelin-overexpressing cancer.

The cancer may be a solid cancer, for example, a mesothelin-positive or mesothelin-overexpressing solid cancer. For example, the solid cancer includes one selected from the group consisting of esophageal cancer, breast cancer, triple-negative breast cancer (TNBC), gastric cancer, cholangiocarcinoma, pancreatic cancer, colon cancer, lung cancer, thymic carcinoma, mesothelioma, ovarian cancer, endometrial cancer, cervical cancer, uterine serous carcinoma (USC), non-small cell lung cancer, and pediatric acute myeloid leukemia (AML),and is not limited thereto.

The pharmaceutical composition may include 10 wt% to 95 wt% of cells, which are active ingredients, based on the total weight of the pharmaceutical composition. In addition, the pharmaceutical composition of the present disclosure may further include one or more active ingredients that exhibit the same or similar functions in addition to the active ingredients.

The dosage of the cells may be adjusted depending on various factors, such as the type of disease, the severity of the disease, the type and content of the active ingredient and other ingredients contained in the pharmaceutical composition, the type of dosage form, and the patient's age, weight, general health, gender and diet, and time of administration, route of administration, treatment period, and drugs used simultaneously. However, for a desirable effect, the effective amount of cells included in the pharmaceutical composition according to the present disclosure may be $1 \times 10^5$ cells/kg to $1 \times 10^{11}$ cells/kg. In this regard, the administration may be carried out once a day, or several times a day. Effective amounts of cells or pharmaceutical compositions presented herein can be determined empirically without undue experimentation.

The pharmaceutical composition may be a preparation having a formulation suitable for the purpose, according to a conventional method in the pharmaceutical field. In addition, the pharmaceutical composition may be formulated and administered in a unit dosage form suitable for administration into the patient's body according to a conventional method in the pharmaceutical field. In addition to the active ingredient, the pharmaceutical preparation may further include one or more pharmaceutically acceptable inert carriers, for example, in the case of injections, a preservative, an analgesic agent, a solubilizer, or a stabilizer, in the case of preparations for topical administration, a base, an excipient, a lubricant, or a preservative.

In addition, the cells or the pharmaceutical compositions including the same may be administered to a subject by various methods known in the art, for example, intraperitoneal administration, intravenous administration, intramuscular administration, subcutaneous administration, intradermal administration, oral administration, topical administration, intranasal administration, intrapulmonary administration, intrarectal administration, etc., but is not limited thereto.

Advantageous Effects of Disclosure

An anti-mesothelin chimeric antigen receptor according to one aspect exhibits a specific binding ability to mesothelin with increased affinity for mesothelin, and can be usefully for the prevention or treatment of cancer in which mesothelin is overexpressed. Brief Description of Drawings

FIG. 1 is a diagram showing polymerase chain reaction (PCR) conditions used to clone affinity-improved antibody candidates.

FIG. 2 is a diagram showing the tertiary structure of MSLN 34, which is an antibody calculated in Discovery studio 2021.

FIG. 3 is a diagram showing a representative docking model for each cluster among various docking models confirmed through Discovery studio.

FIG. 4 is a diagram showing the structure of the docking model for the finally selected MSLN and MSLN34 antibodies.

FIG. 5 is a diagram showing SDS-PAGE results of 7 mutant antibody candidates (D31K, D31R, D31W, D31L, S192F, S192R, and Y228R) including wild type (WT) after purification.

FIGS. 6 to 8 show the results of measuring the ELISA-based affinity for mesothelin with respect to WT and five types of mutant antibodies (D31W, D31L, S192F, S192R, and Y228R).

FIGS. 9 and 10 are diagrams confirming the affinity for mesothelin of mutants including WT, single mutants of D31L and S192R, and mutation of mutant D31L and mutant S192R, of MSLN34.

FIG. 11 shows the amino acid sequences of the scFv of the anti-MSLN chimeric antigen receptor vector with increased and improved affinity including WT, single mutants of D31L and S192R and mutation of mutant D31L and mutant S192R of MSLN 34.

FIGS. 12 and 13 show the characterization of mutant MSLN CAR-T with increased affinity confirmed through Batch #1 experiment.

FIGS. 14 and 15 show the characterization of mutant MSLN CAR-T with increased affinity confirmed through Batch #2 experiment.

FIG. 16 is a diagram confirming the cytotoxic effect of mutant MSLN CAR-T with increased affinity on mesothelioma and ovarian cancer cells through Batch #1 experiment using calcein release assay (calcein-AM).

FIG. 17 is a diagram confirming the cytotoxic effect of mutant MSLN CAR-T with increased affinity on mesothelioma and ovarian cancer cells through Batch #2 experiment using calcein release assay (calcein-AM).

FIG. 18 is a diagram from which the *in vitro* pancreatic cancer cell cytotoxic effect of anti-MSLN-CAR-T cells with increased and improved affinity was confirmed through an incucyte based real-time cytotoxicity assay.

FIG. 19 is a diagram showing the change in body weight of a pancreatic cancer animal model after CAR-T cell treatment.

FIG. 20 is a diagram showing change in tumor volume in a pancreatic cancer animal model after CAR-T cell treatment.

FIG. 21 shows a naked eye view of a pancreatic cancer animal model on day 49 after CAR-T cell treatment.

FIG. 22 is a naked eye view of the separated tumor of a pancreatic cancer animal model on day 49 after CAR-T cell treatment.

FIG. 23 is a diagram showing the tumor weight of a pancreatic cancer animal model after CAR-T cell treatment.

FIG. 24 is a diagram showing the results of immunohistochemical staining of tumor sections of a pancreatic cancer animal model after CAR-T cell treatment (magnification: 5x). Staining was performed using human CD3ε antibody.

FIG. 25 is a diagram showing the results of immunohistochemical staining of tumor sections of a pancreatic cancer animal model after CAR-T cell treatment (magnification: 20x). Staining was performed using human CD3ε antibody.

Mode of Disclosure

Hereinafter, one aspect will be described in more detail through examples. However, these examples are for illustrative purposes only, and the scope of an aspect is not limited to these examples. The embodiments of an aspect provide a more thorough understanding of an aspect to a person with average knowledge in the art.

<u>Materials and Methods</u>

**1. Materials, equipment, and experimental methods used in experiments to construct antibodies having high affinity for mesothelin**

**1.1 Materials and equipment**

For an experiment to construct an anti-MSLN chimeric antigen receptor with improved affinity for mesothelin, MSLN34 antibody, which is an anti-mesothelin antibody, was used as a control.

In addition, specific reagents and equipment used in experiments are shown in Tables 2 and 3 below.

Table 2

| Reagent name | Manufacturing company | Cat. No. |
|---|---|---|
| Top10F' chemically competent *E. coli* | Invitrogen | 44-0300 |
| JUMBO HIT-DH5α | R.B.C. | TH617-J80 |
| Gibson assembly® | N.E.B. | E2611 |
| LB broth miller | EMD Millipore Corp. | 71753-6 |
| Ampicillin sodium salt | Sigma-Aldrich | A9518-2 |
| Isopropylβ-D-1-thiogalactopyranoside | GenDEPOT | I0355-005 |
| Sucrose | Sigma-Aldrich | S9378-5KG |
| Strep- Tactin® XT 4Flow® resin | IBA | 2-5010-025 |
| 10X PBS | Bio world | HP2007-1 |
| 10X Buffer BXT | IBA | 2-1042-025 |
| Human Mesothelin/MSLN (296-580) | Acrobiosystems | MSN-H5223 |
| ELISA plate | Costar | 3690 |
| Anti-strep HRP | abcam | Ab191338 |
| TMB substrate | Sigma | T0440 |
| 10X PBST | LPS solution | CBP007T |

[Table 3]

| Equipment name | Manufacturing company | Cat. No. |
|---|---|---|
| Discovery studio 2021 system | Dassault Systems Biovia corp | Dassault Systems Biovia corp. |
| Shaking incubator | DAIHAN Scientific | WIS-20R |

(continued)

| Equipment name | Manufacturing company | Cat. No. |
|---|---|---|
| Centrifuge | Thermo fisher Scientific | Sorvall Micro21R |
| Centrifuge | Thermo fisher Scientific | Sorvall ST16R |
| Microplate reader | Molecular sevices | VERSA max |

**1.2 Design of antibody with improved affinity for mesothelin**

To develop antibodies with improved affinity for mesothelin, Discovery studio 2021 was used.

Specifically, the tertiary structure of MSLN34 antibody, which is an anti-mesothelin antibody, was calculated using the Model Antibodies function of Discovery studio 2021. Using the docking function (ZDOCK) of Discovery studio 2021, an antigen-antibody docking model was calculated, and in consideration of a binding force, etc., the best docking model was selected from among the calculated docking models. Next, an affinity-improved antibody was designed using the selected docking model using the Mutation (binding energy) function of Discovery studio 2021.

**1.3 Selection of antibodies with improved affinity for MSLN**

**1.3.1 Cloning of affinity-improved antibody candidates**

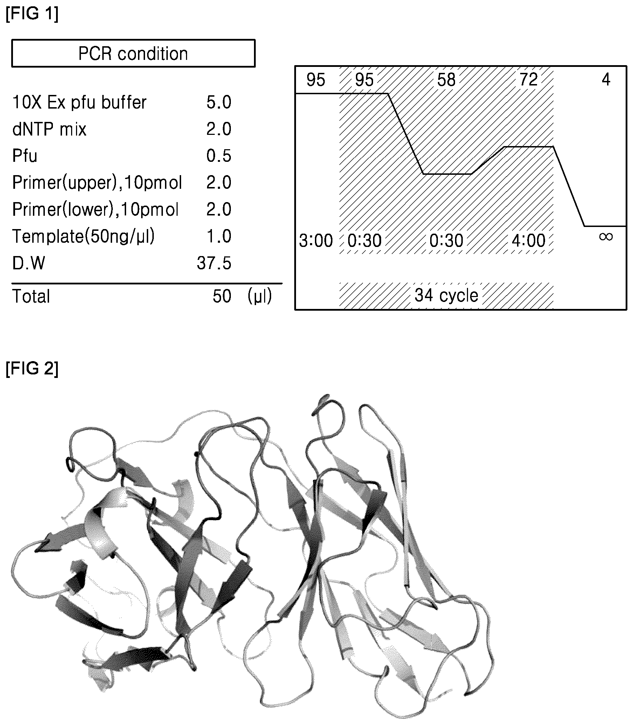

Based on the mutation information calculated in Discovery studio 2021, primers were designed to enable cloning into a gene sequence that can encode the designed amino acid. The sequences of primers used for mutation cloning are shown in Table 4 below, and PCR conditions used are shown in FIG. 1.

[Table 4]

| Primer | Sequence (5'->3') | SEQ ID NO |
|---|---|---|
| D31K_F | AAATATGGTATGCACTGGGTTCG | 5 |
| D31K_R | ATACCATATTTAGAGAAAGTAAAACCCGAG | 6 |
| D31W_F | CTCTTGGTATGGTATGCACTGGGTTCG | 7 |
| D31W_R | ATACCATACCAAGAGAAAGTAAAACCCGAG | 8 |
| D31L_F | CTCTCTGTATGGTATGCACTGGGTTCG | 9 |
| D31L_R | ATACCATACAGAGAGAAAGTAAAACCCGAG | 10 |
| D31R_F | CTCTCGTTATGGTATGCACTGGGTTCG | 11 |
| D31R_R | ATACCATAACGAGAGAAAGTAAAACCCGAG | 12 |
| S192F_F | GCAGTTTGGTGTACCGTCCCGT | 13 |
| S192F_R | GGTACACCAAACTGCAGAGAGGAAG | 14 |
| S192R_F | GCAGCGTGGTGTACCGTCCCGT | 15 |

(continued)

| Primer | Sequence (5'->3') | SEQ ID NO |
|---|---|---|
| S192R_R | GGTACACCACGCTGCAGAGAGGAAG | 16 |
| Y228R_F | CGCTCTTTTCCGTTTACGTTCGG | 17 |
| Y228R_R | AAACGGAAAAGAGCGAGATTGCTGAC AAT | 18 |

The PCR product of each mutant was cloned using the Gibson assembly method and transformed into *E. coli* DH5α to obtain a single clone. The amino acid sequence of the final candidate was confirmed through nucleotide sequence analysis through sequencing.

**1.3.2 Production of mutant antibody candidates**

The cloned gene was transformed into *E.coli* TOP10F', which is a strain for protein expression, spread on LB solid medium including the antibiotic ampicillin, and cultured at 37 °C for 20 hours to obtain a transformant. For pre-culture, colonies of the transformants were cultured with shaking at 37 °C for 16 hours in LB medium including 10 mL of ampicillin, and 5 mL of cultured cells were inoculated into LB medium including 500 mL of ampicillin. When the cell density (O.D600) reached 0.6 or higher by culturing at 37 °C, 0.5 mM IPTG was added, and the cells were expressed at 30 °C and harvested 16 hours later. The harvested *E. coli* was caused to react in 1xTES buffer (50 mM tris-HCl, 1 mM EDTA, 20% sucrose, pH 8.0) for 1 hour, and then additionally reacted in 0.2xTES buffer for 1 hour to be completely lyzed. The lyzed *E. coli was* centrifuged (15,000 rpm, 40 min, 4 °C) to recover the supernatant and flowed through a column equilibrated with 1xPBS to bind to a resin inside. After washing the resin with 1xPBS, an antibody was recovered with 1xBXT buffer. The recovered protein was concentrated using a centrifugal filter (3 kDa).

**1.3.3 ELISA-based affinity measurement**

30 μL of 1 μg/mL MSLN antigen was dispensed into each well of a 96-well ELISA plate coated with polystyrene and caused to react at 4 °C for 16 hours. After the reaction, the MSLN antigen was removed therefrom, and the cells were treated with a blocking solution of 5% MPBS (5% w/v skimmed milk powder in PBS), and 1 mg/mL of antibody was diluted at 1/3, 1/9, 1/27, 1/81, and 1/243 and reacted at room temperature for 1 hour. After washing again with PBST buffer 4 times, the result was reacted with HRP-anti-strep at 37 °C for 1 hour. After washing with PBST buffer four times, the result was reacted with a TMB substrate at room temperature for 8 minutes, and the reaction was stopped with 2N $H_2SO_4$. Absorbance values were confirmed at OD 450 nm using a micro plate reader.

**2. Materials and equipment used in the experiment to confirm the cytotoxic ability of CAR-T anticancer cells including the newly discovered MSLN34 variant scFv with high affinity**

**2.1 Materials and cell lines, reagents and equipment used**

The cytotoxic ability of CAR-T carrying MSLN34 scFv variants (MSLN34-D31L, MSLN34-S192R, and MSLN34-D31L/S192R) obtained through in silico-based affinity maturation for the scFv of MSLN34, an anti-mesothelin antibody, against pancreatic cancer, mesothelioma, and ovarian cancer cell lines, was performed, and the materials used in the experiment were as follows.

A lentiviral vector (pLV) was used as a vector expressing anti-MSLN CAR, and the cell lines used are shown in Tables 5 to 7 below.

[Table 5]

| Cell line information | 293T |
|---|---|
| Organism | Homo sapiens, human |
| Tissue | Embryonic kidney |
| Disease | Normal cell |
| Properties | Adherent |

(continued)

| Cell line information | 293T |
|---|---|
| Culture conditions | DMEM, 10% FBS, 1% penicillin and streptomycin, 5% $CO_2$, 37°C |
| Supplier | ATCC (CRL-3216) |

[Table 6]

| Cell line information | AsPC-1/GFP |
|---|---|
| Organism | Homo sapiens, human |
| Tissue | Pancreas |
| Disease | Adenocarcinoma |
| Properties | Adherent |
| Culture conditions | RPMI1640, 10% FBS, 1% penicillin and streptomycin, 5% $CO_2$, 37°C |
| Supplier | In-house |

[Table 7]

| Cell line information | NCI-H2052 |
|---|---|
| Organism | Homo sapiens, human |
| Tissue | Lung |
| Disease | Mesothelioma |
| Properties | Adherent |
| Culture conditions | RPMI1640, 10% FBS, 1% penicillin and streptomycin, 5% $CO_2$, 37°C |
| Supplier | ATCC (CRL-5915) |

[Table 8]

| Cell line information | OVCAR-3 |
|---|---|
| Organism | Homo sapiens, human |
| Tissue | Ovary |
| Disease | Adenocarcinoma |
| Properties | Adherent |
| Culture conditions | RPMI1640, 20% FBS, 1% penicillin and streptomycin, 5% $CO_2$, 37 °C |
| Supplier | ATCC (HTB-161) |

Information on the lentivirus production vector used is shown in Table 9 below.

[Table 9]

| No. | Category | Vector type | Cat. No. | Manufacturi ng company | Remark |
|---|---|---|---|---|---|
| 1 | Packaging vector | pMDLg/pRRE | 12251 | Addgene | - |
| 2 | Packaging vector | pRSV-Rev | 12253 | Addgene | - |
| 3 | Packaging vector | pMD2.G | 12259 | Addgene | - |
| 4 | Expression vector | pLV-MSLN | - | KBIO | 2nd G. KBIO CAR |

Additionally, other reagents and materials are shown in Table 10 below.

[Table 10]

| Reagent | Cat. No. | Manufacturing company |
|---|---|---|
| RPMI1640 | 11875093 | Gibco |
| Fetal Bovine Serum | 16000044 | Gibco |
| DPBS, 1X | 14190250 | Gibco |
| Trypsin-EDTA (0.05%) | 25300054 | Gibco |
| Penicillin-Streptomycin (10,000 U/mL) | 15140122 | Gibco |
| Protamine sulfate | P3369-10G | Sigma |
| KOD plus mutagenesis kit | SMK-101 | Toyobo |
| PBMC | CC-2702 | Lonza |
| IL-2 recombinant human protein | CTP0021 | Gibco |
| TransAct bead | 130-128-758 | MACS |
| APC CD3 | 555342 | B.D. |
| Biotin-MSLN | MSN-H8223 | Acrobiosystems |
| PE anti-biotin | 130-110-951 | MACS |
| Sucrose | S1030 | Biosesang |
| APC Mouse Anti-Human CD3 | 555342 | B.D. |
| NucleoBond® Xtra Maxi EF plasmid DNA purification | 740424.5 | MACHEREY-NAGEL |
| NucleoBond® Xtra Midi plasmid DNA purification | 740410.50 | MACHEREY-NAGEL |
| Calcein-AM | C-1439 | Invitrogen |

Additionally, the equipment used is shown in Table 11.

[Table 11]

| Equipment | Model number | Manufacturing company |
|---|---|---|
| Biological Safety Cabinet | 1367 | ThermoFisher |
| Centrifuge | 5810R | Eppendorf |
| $CO_2$ incubator | Galaxy 170S | Eppendorf |
| CountessTM II Automated Cell Counter | AMQAX1000 | ThermoFisher |
| Ultracentrifuge | Optima XE-100 | Beckman Coulter |
| Incucyte | Incucyte zoom | Essen bioscience |
| FACSCantoTM II | 338960 | B.D. |
| NanoDrop™ 2000 Spectrophotometer | ND-2000 | ThermoFisher |
| Multi-mode microplate reader | FilterMax F5 | Molecular devices |
| FLOWJO Single Cell Analysis Software V10 | 663335 | FlowJo, LLC. |
| GraphPad Prism (Ver. 9) | - | GraphPad Software |

**2.2 Construction of pLV lentiviral expression vector expressing anti-MSLN CAR**

A vector was constructed using MSLN34-D31L, MSLN34-S192R, and MSLN34-D31L/S192R, which are MSLN34 scFv-based affinity maturated scFv sequences, obtained from the artificial intelligence structural design team of the New Drug Development Support Center, using the following method. Affinity maturated anti-MSLN scFv-loaded CAR vectors (MSLN34-D31L CAR, MSLN34-S192R CAR, and MSLN34-D31L/S192R CAR) based on MSLN34 CAR vector(REP-RD21-011) made in the second generation KBIO CAR vector were constructed using the KOD plus mutagenesis kit. The construction method was performed according to the manual of the KOD plus mutagenesis kit. The constructed vector was confirmed to have no abnormalities in the entire anti-MSLN scFv gene sequence through gene sequence analysis.

**2.3 Plasmid DNA extraction for lentivirus production**

Genes were introduced such that lentivirus packaging plasmids (pMDLg/pRRE, pRSV-Rev, pMD2.G) and pLV MSLN DNA vector were introduced into *E. coli* (DH5$\alpha$) bacterial strain using heat shock transformation. Plasmid DNA was extracted according to the NucleoBond® Xtra® Maxi EF kits and NucleoBond® Xtra Midi plasmid DNA purification manual. The concentration and purity of the extracted plasmid DNA were measured using a NanoDrop™ 2000 spectrophotometer.

**2.4 Lentivirus production and concentration/purification**

293T cells were seeded in a 100 mm cell culture dish at a concentration of 6.0 x 10$^6$ cells/dish and cultured for 1 day. pMDLg/pRRE, pRSV-Rev, and pMD2.G DNA, which are third-generation lentivirus packaging plasmids, and MSLN CAR vector DNA were each diluted in Opti-MEM according to a set ratio and then transformed using Lipofectamine 3000. Transformation was performed according to the Lipofectamine 3000 user manual. The lentivirus culture medium after production was completed was purified/concentrated using a 20% sucrose gradient purification method and finally stored at -80 °C.

**2.5 Measurement of Infectious titer of lentivirus using FACS analysis**

HeLa cells were seeded in a 6-well plate at a concentration of 1.5 x 10$^5$ cells/well and cultured for 1 day. The next day, lentivirus and polybrene whose titer was to be measured, were added to HeLa cells at a final concentration of 8 $\mu$g/mL and added to each well, and transduction was performed thereon. Cells were obtained for FACS analysis 48 hours after transduction.
Using recombinant MSLN protein as an antigen, the number of cells bound to the anti-MSLN scFv antibody region was measured by FACS analysis. The infectious titer conversion formula is as follows.

Transducing Unit (TU)/mL = [(Seeded cells #) x (Frequency PE+ cells) x 1000]/($\mu$L of lentivirus vector)

**2.6 MSLN CAR-T construction**

**2.6.1 T cell activation (activation) performed**

Human PBMC was dissolved and then diluted in 9 mL of T cell culture medium (RPMI-1640 medium + 10% FBS + 1% penicillin-streptomycin + IL-2 200 U/mL) and centrifuged for 7 minutes at room temperature and 300 g. Afterwards, the supernatant was removed and re-suspended in 10 mL of new T cell culture medium. The differentiation procedure from human PBMC to T cells was performed according to the manual provided by the manufacturer of TransAct bead reagent.

**2.6.2 MSLN CAR transduction**

One day after starting T cell activation, all activated T cells in culture were harvested and centrifuged at 300 g for 7 minutes at room temperature. Activated T cells were prepared to enter a new 24-well plate at a concentration of 5.0 x 10$^5$ cells/well, and culture was performed in a final volume of 0.5 mL. For each experimental group, lentivirus was added at an MOI of 5 based on the infectious titer, and protamine sulfate was added at a final concentration of 1 $\mu$g/mL and seeded in a new 24 well plate. A 24-well plate was subjected to spin infection at 300 g, 32 °C for 90 minutes, and then cultured in an incubator at 37 °C and 5% CO$_2$. The next day, all T cells were harvested and centrifuged at 300 g for 7 minutes, and the supernatant was removed therefrom, and new culture medium was added and cultured.

**2.6.3 MSLN CAR-T characterization**

Cultured MSLN CAR-T cells were adjusted to be 1 x 10$^6$ cells using a cell counter. After washing the cells using washing buffer (PBS + 2% FBS), biotin-MSLN antigen was added and stored in a refrigerator for 20 minutes. After washing the cells again using washing buffer, PE-biotin antibody and APC-CD3 antibody were added and refrigerated for 20 minutes while blocking light. Finally, after washing the cells using washing buffer, the cells were re-suspended in 100 $\mu$L of washing buffer and finally FACS analysis of differentiated CAR-T was performed thereon.

**2.7 MSLN CAR-T in vitro efficacy evaluation: Calcein release assay**

**2.7.1 Preparation of target cells stained with Calcein-AM**

The required amount of cells were placed in a 1.5 mL tube, and Calcein-AM was added thereto at a final concentration of 10 $\mu$g/mL, and staining was performed thereon for 1 hour at 37 °C. Centrifugation was performed at 1,200 rpm for 5 minutes at room temperature, and the cells were washed three times using 1 mL of culture medium. Calcein-AM-stained cells were seeded in a 96-well plate (R type) at a concentration of 1.0 x $10^4$ cells/well.

**2.7.2 Preparation of effector cells (MSLN CAR-T)**

Starting with E:T = 10:1, which is the ratio of effector cells that would react with target cells, a 2-fold serial dilution method was performed. A total of 4 E:T ratios were used, and according to the expression ratio of each CAR, the calculation was corrected based on the number of CAR-expressing T cells. However, in the case of mock T cells, the number of cells was calculated to be the same as the greatest number of cells when corrected for the CAR expression rate. The spontaneous release of Calcein-AM was treated with RPMI-1640 medium. The maximum release of Calcein-AM was treated with 2% Triton X-100.

**2.7.3 Calcein release analysis performed**

Target cells and effect cells were mixed and co-cultured for 4 hours at 37 °C and at 5% $CO_2$. Centrifugation was performed for 5 minutes at room temperature and at 100 g. To measure the amount of Calcein-AM leaked out of the cell due to apoptosis, 100 $\mu$L of co-culture culture medium was transferred to a black optical plate (F type). The values were measured in the wavelength range including the excitation wavelength of 485 nm and the emission wavelength of 530 nm using a microplate reader capable of measuring fluorescence. The calculation formula to obtain the cytotoxic effect value is as follows.

% of Specific Lysis = [(Test release - Spontaneous release)/(Maximum release - Spontaneous release)] x 100%

**2.8 MSLN CAR-T *in vitro* efficacy evaluation: Incucyte based real-time cytotoxicity assay**

**2.8.1 Target cell preparation**

AsPC-1/GFP cells were seeded at 100 $\mu$L each in a 96 well plate at 1 x $10^4$ cells/well.

**2.8.2 Effector cell preparation**

This test was performed on Mock T (Non transducing T) and MSLN CAR-T (MSLN34, MSLN34-D31 L, MSLN34-S192R, and MSLN34-D31L/S192R) cells. The ratio of effector cells to react with target cells was adjusted to be E:T = 0.5:1 and corrected based on the number of CAR-expressing T cells according to the expression ratio of each CAR. However, in the case of mock T cells, the number of cells was calculated to be the same as the greatest number of cells when corrected for the CAR expression rate.

**2.8.3 Real-time cytotoxicity assay**

Target cells and effector cells were mixed and co-cultured. Incucyte was set to measure GFP every 3 hours for 72 hours in a 96 well plate, and then the plate in co-culture was loaded. After all incucyte GFP measurements were completed, the GFP measurements were analyzed.

**2.9 Experimental data and statistical analysis**

For the incucyte analysis and calcein release analysis, experimental data were obtained using four independent E + T co-culture wells per experimental group (Tetra-plicated assay). All experimental data were plotted using GraphPad Prism 9.0 software. The statistical significance of all experimental data was determined using the statistical analysis tool Two-Way ANOVA (Full model, Tukey, 95% confidence interval) included in GraphPad Prism 9.0 software. (ns, P > 0.05; *, P $\leq$ 0.05; **, P $\leq$ 0.01; ***, P $\leq$ 0.001)

**3. Materials, equipment, and experimental methods used in the experiment to confirm the anticancer effect of CAR-T including the newly discovered MSLN34 scFv with high affinity on an animal model of pancreatic cancer**

**3.1 Production of pancreatic cancer (AsPC-1) animal model**

**3.1.1 Mice breeding**

To create a pancreatic cancer animal model, 6-week-old (15.0 g to 25.0 g) male specific pathogen-free (SPF) mice of the NOG (NOD/Shi-*scid*/IL-2R$\gamma^{null}$) strain were used. The mice breeding and related tests were conducted under conditions including a temperature of 22 $\pm$ 2 °C, relative humidity of 50 $\pm$ 10%, ventilation frequency of 10 times/hr to 20 times/hr, and lighting time of 12 hours (lights on from 8 a.m. to 8 p.m.), and illumination intensity of 150 Lux to 300 Lux. This study was conducted in the animal room of the Osong Advanced Medical Industry Promotion Foundation Laboratory Animal Center. The mice were allowed to consume food and water freely. The mice-related tests were conducted in compliance with the Osong Advanced Medical Industry Promotion Foundation Laboratory Animal Management Committee regulations.

**3.1.2 Cell culture and transplantation**

To create a pancreatic cancer animal model, the AsPC-1 cell line was used. The cell line was tested for Mycoplasma pneumoniae, Murine coronavirus (Mouse hepatitis virus, MHV), and Murine respirovirus (Sendai virus, SeV) and used after being confirmed negative. The cell line was cultured in a $CO_2$ incubator at 37 °C and 5% $CO_2$ using a medium including RPMI-1640, 10% FBS, and 1% P/S (penicillin/streptomycin). The AsPC-1 cells were subcutaneously implanted at 200 uL each into mice after the cell concentration thereof was adjusted using PBS.

**3.2 Composition of pancreatic cancer animal model test group**

The pancreatic cancer animal model produced above was divided into groups based on tumor size by random distribution, and test groups were constructed as shown in the table below.

[Table 12]

| Group | n= | Cell line (cell count/cell) | Test substance | Volume (CAR-Ts/Marie ) | Route of administration | Volume |
|---|---|---|---|---|---|---|
| G1 | 5 | | HBSS | - | | |
| G2 | 5 | | Mock ($1.5 \times 10^6$) | - | | |
| G3 | 5 | AsPC-1 ($5 \times 10^6$) | MSLN34 (WT) | $1.5 \times 10^6$ | IV | 200 uL |
| G4 | 5 | | MSLN34(WT) | $0.5 \times 10^6$ | | |
| G5 | 5 | | MSLN34-D31L | $1.5 \times 10^6$ | | |
| G6 | 5 | | MSLN34-D31L | $0.5 \times 10^6$ | | |

For individual identification of the test groups, the ear-punch method was used during the test period, and identification cards for each group were attached to the breeding boxes. After group separation, the test substance was administered as a single dose through intravenous (IV).

**3.4 Body weight and tumor size measurements**

The body weight and tumor size of the test group were measured twice a week from the start of administration. The body weight on the starting day of administration (Day 0) was used as the standard, and changes in body weight were identified until the end date of the test. Body weight (%) was calculated using the formula below.

$$\text{Body weight (\%)} = (\text{Body weight / Body weight at Day 0}) \times 100$$

Tumor size ($mm^3$) was calculated by measuring the short axis (A) and long axis (B) of the tumor using calipers and using the following formula.

$$\text{Tumor volume (mm}^3) = ([A(mm)]^2 \times B(mm)) / 2$$

### 3.5 Autopsy

Anesthesia was induced by intraperitoneal injection of Zoletil™ (50 mg/kg) and Rompun (10 mg/kg), the abdominal cavity was opened, blood was collected from the abdominal vena cava, and euthanasia was performed by exsanguination. Serum was isolated from blood, and then stored frozen (below -80 °C). A portion of the isolated tumor was stored frozen (-80 °C or lower), and a portion thereof was fixed in a fixative (10% neutral formalin) and then subjected to histopathology. Slides were prepared and Hematoxylin and eosin (H&E) staining was performed. Stained slides were photographed using PANNORAMIC SCAN II (3DHISTECH, Hungary) and analyzed with 3DHISTECH software.

### 3.6 Data analysis

Statistical comparison of data was analyzed using SPSS 10.1. Data were expressed as mean $\pm$ standard deviation (SD), and analysis was performed by one-way ANOVA followed by Tukey's post hoc analysis for multiple comparisons (*: $p<0.05$, **: $p<0.01$, ***: $p<0.001$ vs. vehicle control (G1), #: $p<0.05$, ##: $p<0.01$, ###: $p<0.001$ vs. mock cell treated group (G2)).

### Conclusion

**Experimental Example 1: Calculation of docking model of antigen (MSLN)-antibody (MSLN34)**

First, the tertiary structure of MSLN34, which is an antibody that binds specifically to the antigen mesothelin, was calculated. MSLN34 was calculated through the homology model function of Discovery studio 2021, and the calculated structure is shown in FIG. 2. As can be seen in FIG. 2, the CDR region of the antibody is expressed in pink (light chain) and blue (heavy chain).

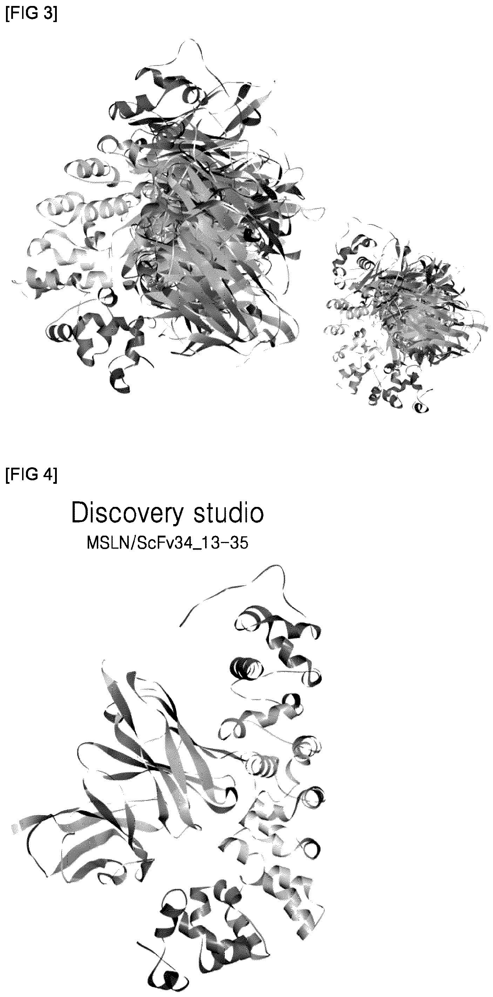

Next, the antigen-antibody docking model for mesothelin and MSLN34 was calculated using the ZDOCK program in Discovery studio 2021. As a result, as seen in FIG. 3, various docking models binding to the curved inner surface of MSLN from the N-terminus to the C-terminus could be seen, and various binding positions could be confirmed. As a result of Discovery studio 2021's docking program, approximately 2,000 docking models were calculated, and the structure of about approximately 200 models was identified in order of highest binding force prediction value, and the docking model in FIG. 4 was finally selected in consideration of the presence or absence of specific binding between mesothelin and MSLN34.

As confirmed in FIG. 4, the selected docking model binds to amino acids 385th to 569th of mesothelin, and these binding sites were confirmed to be the positions for which the most docking models were calculated, in the docking results of Discovery studio 2021. In addition, it was confirmed that the amino acid involved in the binding of MSLN34 to the target mesothelin uses all six variant regions, indicating that the structure thereof forms a stable binding.

**Experimental Example 2: Selection of MSLN 34 antibody with increased affinity**

**2.1 Design of mutant candidates with increased affinity based on the structure of the *in silico* model**

An experiment was designed to identify an antibody capable of increasing a binding force based on the docking model selected by Experimental Example 1 above. It was calculated using the Mutagenesis (Binding) function in Discovery studio 2021, and the calculation results are shown in Table 13 below.

[Table 13]

| Cluster-3 calculate mutation energy (Binding) | | | | | | |
|---|---|---|---|---|---|---|
| Mutation | Mutation Energy | Effect of Mutation | VDW term | Electrostatic term | Entropy term | Non-polar Term |
| B:ASP31>AR G | -3.24 | STABILIZI NG | -4.66 | -4.26 | 1.53 | 0 |
| B:ASP31>LY S | -3.22 | STABILIZI NG | -6.14 | -2.55 | 1.4 | 0 |
| B:ASP31>TR P | -3.1 | STABILIZI NG | -4.37 | -2.52 | 0.43 | 0 |
| B:ASP31>LE U | -3.05 | STABILIZI NG | -3.8 | -2.69 | 0.24 | 0 |

(continued)

| Cluster-3 calculate mutation energy (Binding) | | | | | | |
|---|---|---|---|---|---|---|
| Mutation | Mutation Energy | Effect of Mutation | VDW term | Electrostatic term | Entropy term | Non-polar Term |
| B:SER192>A RG | -3.04 | STABILIZI NG | -6.8 | -0.8 | 0.95 | 0 |
| B:ASP31>HIS | -2.73 | STABILIZI NG | -3.89 | -2.17 | 0.38 | 0 |
| B:SER192>P HE | -2.73 | STABILIZI NG | -4.98 | -0.28 | -0.12 | 0 |
| B:ASP31>PH E | -2.62 | STABILIZI NG | -2.83 | -2.68 | 0.17 | 0 |
| B:ASP31 >ILE | -2.57 | STABILIZI NG | -2.7 | -2.57 | 0.08 | 0 |
| B:TYR102>A RG | -2.46 | STABILIZI NG | -4.6 | -1.89 | 0.98 | 0 |
| B:ASP31>TY R | -2.44 | STABILIZI NG | -3.15 | -2.49 | 0.48 | 0 |
| B:HIS100>AR G | -2.33 | STABILIZI NG | -3.45 | -1.83 | 0.39 | 0 |
| B:TYR104>A RG | -2.14 | STABILIZI NG | -5.04 | -1.08 | 1.15 | 0 |
| B:TYR228>A RG | -0.87 | STABILIZING | -1.6 | -1.58 | 0.9 | 0 |

As confirmed in Table 13 above, the mutant candidates with the highest affinity in the docking model were selected by sorting the same in descending order of mutation energy.

Specifically, according to the determination that inducing mutation at positions D31, S192, and Y228 most likely increased the affinity of MSLN34 for mesothelin, which is the target in the docking model, mutations were induced in the anti-mesothelin antibody or the antigen-binding fragment thereof. Accordingly, a mutant was designed such that, in the scFv of the anti-mesothelin antibody (MSLN34) (SEQ ID NO: 1), aspartic acid (D) located at the 31st amino acid (1st amino acid of HCDR1) was replaced with lysine (K), tryptophan (W), leucine (L), and arginine (R); serine (S) located at the 192nd amino acid (7th amino acid of LCDR2) to phenylalanine (F) and arginine (R); and tyrosine (located at the 228th amino acid (4th amino acid of LCDR3) was replaced with arginine (R). However, although the mutation energy is low, the positions Y102 and Y104 were excluded because they are amino acids directly involved in binding in the docking model. The mutant candidate sequences of the anti-mesothelin antibody or the antigen-binding fragment thereof with increased affinity designed in this way were analyzed. The sequence of the mutant is a scFv sequence in which amino acids at each position in MSLN34 scFv of SEQ ID NO: 1 are replaced with specific amino acids.

## 2.2 Confirmation of affinity for mesothelin of antibody candidates including single mutants with increased and improved affinity

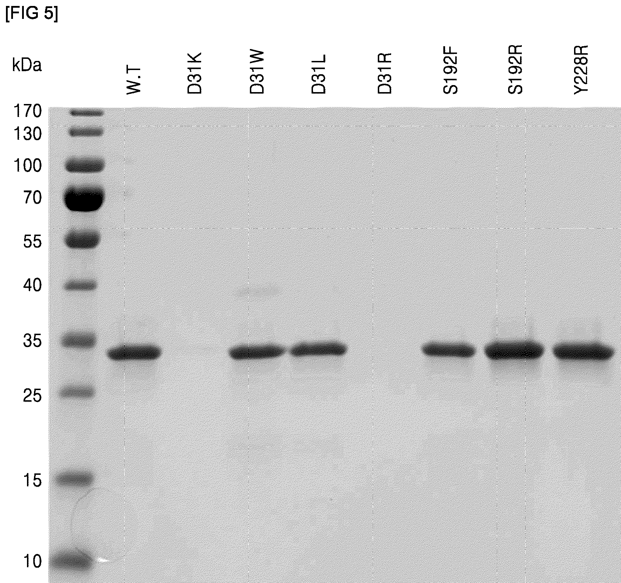

An experiment was performed to confirm whether the antibody candidate of Experimental Example 2.1 could actually be identified as an anti-mesothelin antibody or antigen-binding fragment thereof, with increased and improved affinity. To prepare the anti-mesothelin antibody or the antigen-binding fragment thereof identified in Experimental Example 2.1, the DNA nucleic acid sequence encoding the MSLN34 scFv mutant amino acid sequence was expressed in E. *coli* strain Top10F', and purified by affinity activating using a strep tag, and the purity and production of a total of eight mutant antibody candidates, including wild type (WT), were confirmed on SDS-PAGE gel, and the confirmation results are shown in FIG. 5. As confirmed in FIG. 5, D31K and D31R were not expressed or purified, and WT, D31W, D31L, S192F, S192R, and Y228R were confirmed to be purified with purity of 95% or more.

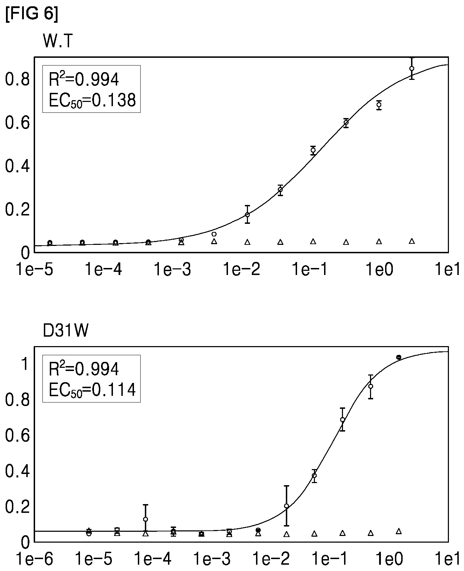

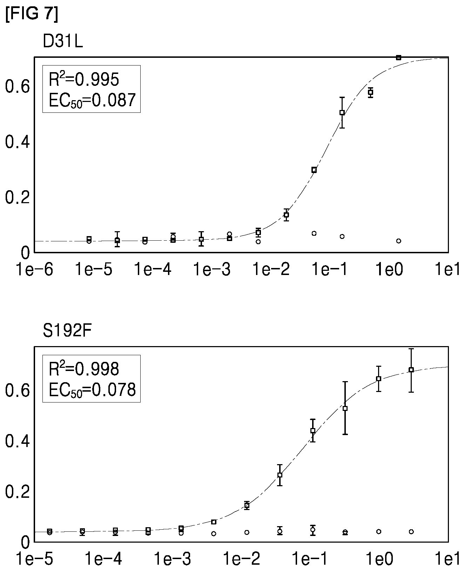

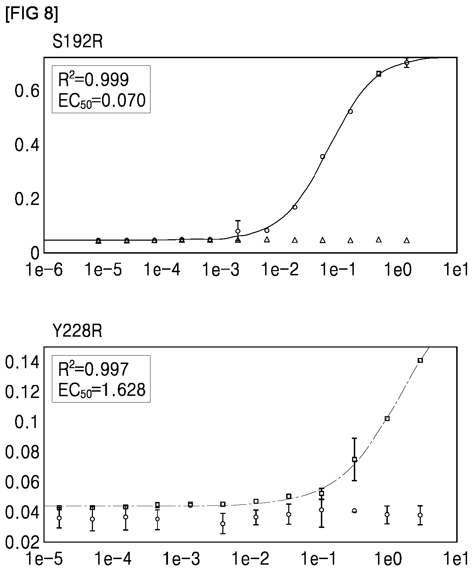

Next, ELISA-based affinity was measured for six types of antibodies, including the purified WT. Mesothelin (MSLN) was attached to a 96 well plate, and the purified antibody was serially diluted 1/3 times and reacted. The affinity graphs are shown in FIGS. 6 to 8. As confirmed in FIGS. 6 to 8, compared to WT, the D31L, S192F, and S192R candidates had smaller $EC_{50}$ values, confirming that the affinity has been increased.

## 2.3 Confirmation of affinity of antibody candidates including double mutants with increased and improved affinity, for mesothelin

In relation to D31L and S192R mutation candidates, of which affinity was increased, from among single mutant antibodies, which was confirmed in the Experimental Example, an experiment was performed to determine whether the affinity for mesothelin would be further increased when a double mutation appears.

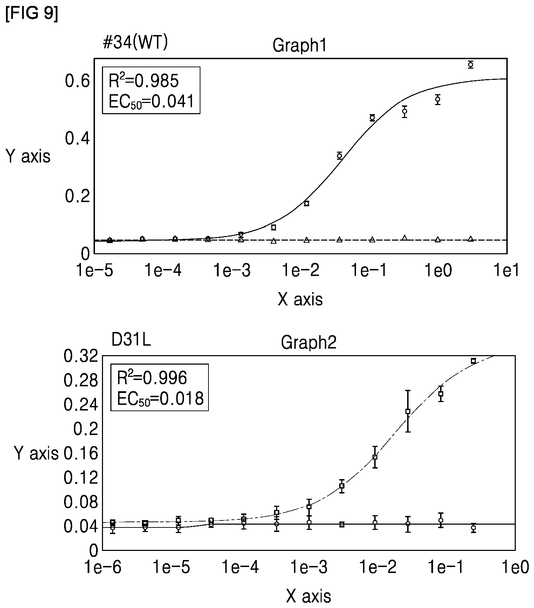

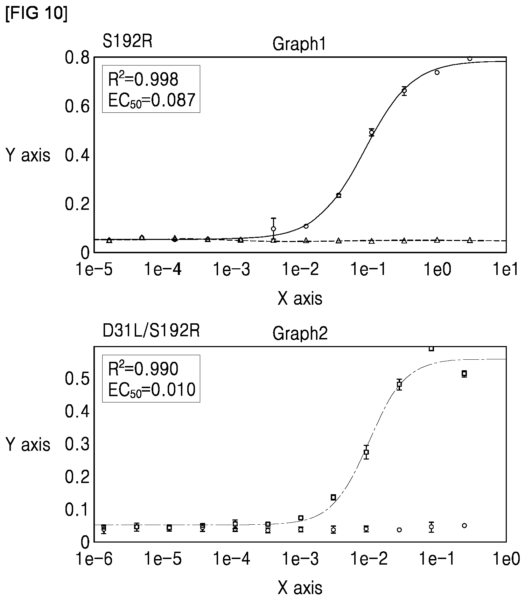

The specific experimental method was performed in the same manner as the affinity measurement of the single mutant antibody in Experimental Example 2.2. The results of confirming the affinity of WT in which no mutation exists, single mutants of D31L and S192R, and mutation of mutant D31L and mutant S192R of MSLN34, are shown in FIGS. 9 and 10. As confirmed in FIGS. 9 and 10, as a result of ELISA-based affinity measurement, it was confirmed that the affinity of the double mutant antibody was significantly increased compared to WT.

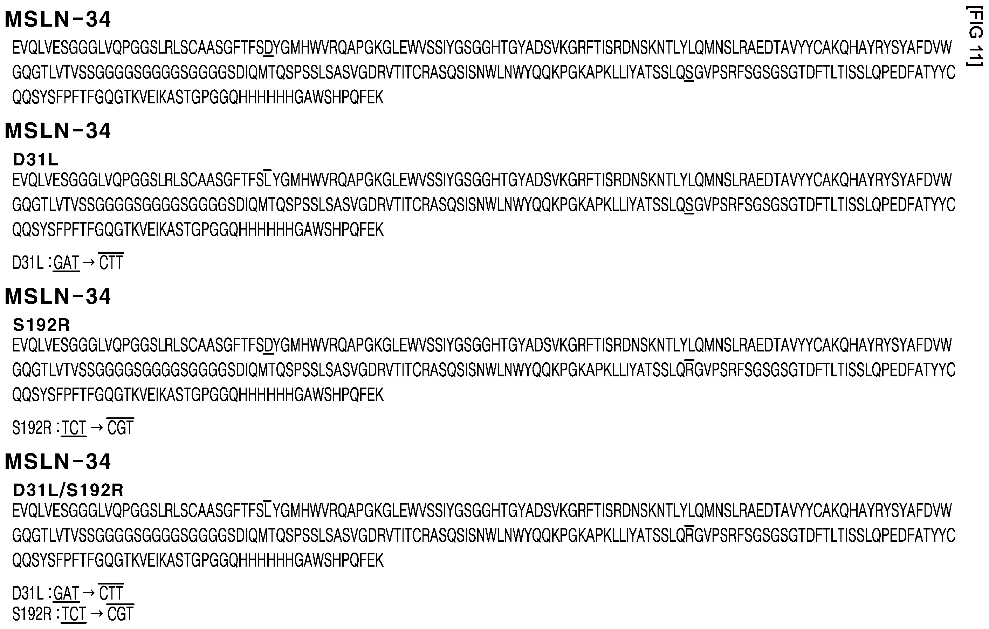

The amino acid sequence of single mutants of D31L (the 1st amino acid of HCDR1 is substituted from D to L) or S192R (the 7th amino acid of LCDR2 is substituted from S to R), and a mutation (D31L/S192R) including mutant D31L and mutant S192R, of MSLN34 scFv, which were confirmed to have improved affinity above. Results are shown in FIG. 11.

In addition, the amino acid sequences of light chain CDR sequence, heavy chain CDR sequence, light chain variable region, and heavy chain variable region of MSLN34 and variants thereof are listed in Table 14 below. In addition, the parts to be substituted in the MSLN34 variant are indicated in bold and underlined.

[Table 14]

| Antibody | Region | Amino acid sequence | SEQ ID No. |
|---|---|---|---|
| MSLN34 WT | HCDR1 | DYGMH | 19 |
| | HCDR2 | SIYGSGGHTGYADSVKG | 20 |
| | HCDR3 | QHAYRYSYAFDV | 21 |
| | LCDR1 | RASQSISNWLN | 22 |
| | LCDR2 | ATSSLQS | 23 |
| | LCR3 | QQSYSFPFT | 24 |
| | VH | EVQLVESGGGLVQPGGSLRLSCAASGFTFSDYGMHWVRQAPGKGLEWVSSIYGSGGHTGYADSVKGRFTISRDNSKNTLYLQMNSLRAEDTAVYYCAKQHAYRYSYAFDVWGQGTLVTVSS | 25 |
| | V.L. | DIQMTQSPSSLSASVGDRVTITCRASQSISNWLNWYQQKPGKAPKLLIYATSSLQSGVPSRFSGSGSGTDFTLTISSLQPEDFATYYCQQSYSFPFTFGQGTKVEIK | 26 |

(continued)

| Antibody | Region | Amino acid sequence | SEQ ID No. |
|---|---|---|---|
| MSLN34 D31L | HCDR1 | LYGMH | 27 |
| | HCDR2 | SIYGSGGHTGYADSVKG | 28 |
| | HCDR3 | QHAYRYSYAFDV | 29 |
| | LCDR1 | RASQSISNWLN | 30 |
| | LCDR2 | ATSSLQS | 31 |
| | LCDR3 | QQSYSFPFT | 32 |
| | VH | EVQLVESGGGLVQPGGSLRLSCAASGFTFSLYGMHWVRQAPGKGLEWVSSIYGSGGHTGYADSVKGRFTISRDNSKNTLYLQMNSLRAEDTAVYYCAKQHAYRYSYAFDVWGQGTLVTVSS | 33 |
| | VL | DIQMTQSPSSLSASVGDRVTITCRASQSISNWLNWYQQKPGKAPKLLIYATSSLQSGVPSRFSGSGSGTDFTLTISSLQPEDFATYYCQQSYSFPFTFGQGTKVEIK | 34 |
| MSLN34 S192R | HCDR1 | DYGMH | 35 |
| | HCDR2 | SIYGSGGHTGYADSVKG | 36 |
| | HCDR3 | QHAYRYSYAFDV | 37 |
| | LCDR1 | RASQSISNWLN | 38 |
| | LCDR2 | ATSSLQR | 39 |
| | LCDR3 | QQSYSFPFT | 40 |
| | VH | EVQLVESGGGLVQPGGSLRLSCAASGFTFSDYGMHWVRQAPGKGLEWVSSIYGSGGHTGYADSVKGRFTISRDNSKNTLYLQMNSLRAEDTAVYYCAKQHAYRYSYAFDVWGQGTLVTVSS | 41 |
| | VL | DIQMTQSPSSLSASVGDRVTITCRASQSISNWLNWYQQKPGKAPKLLIYATSSLQRGVPSRFSGSGSGTDFTLTISSLQPEDFATYYCQQSYSFPFTFGQGTKVEIK | 42 |

(continued)

| Antibody | Region | Amino acid sequence | SEQ ID No. |
|---|---|---|---|
| MSLN34 D31L/S19 2R | HCDR1 | LYGMH | 43 |
| | HCDR2 | SIYGSGGHTGYADSVKG | 44 |
| | HCDR3 | QHAYRYSYAFDV | 45 |
| | LCDR1 | RASQSISNWLN | 46 |
| | LCDR2 | ATSSLQR | 47 |
| | LCDR3 | QQSYSFPFT | 48 |
| | VH | EVQLVESGGGLVQPGGSLRLSCAASGFTFSLYGMHWVRQAPGKGLEWVSSIYGSGGHTGYADSVKGRFTISRDNSKNTLYLQMNSLRAEDTAVYYCAKQHAYRYSYAFDVWGQGTLVTVSS | 49 |
| | VL | DIQMTQSPSSLSASVGDRVTITCRASQSISNWLNWYQQKPGKAPKLLIYATSSLQRGVPSRFSGSGSGTDFTLTISSLQPEDFATYYCQQSYSFPFTFGQGTKVEIK | 50 |

## Experimental Example 3: Construction of anti-MSLN chimeric antigen receptor with increased and improved affinity

### 3.1 Cloning of anti-MSLN-CAR lentivirus vector

To construct a chimeric antigen receptor containing the improved anti-mesothelin antibody or the antigen-binding fragment identified in Experimental Example 2 above, the anti-MSLN-CAR lentivirus vector was cloned.

As a vector, the second-generation CAR lentiviral vector (pLV lentiviral vector) system held by the New Drug Development Support Center, which consists of pMDLg/pRRE (addgene) encoding gag/pol, the envelope plasmid pRSV-Rev (addgene) encoding the Rev protein, and the envelope plasmid pMD2.G (addgene) encoding the VSV-G protein, was used.

First, gene cloning was performed on MSLN34 scFv (antigen-binding domain) and mutants thereof, which were confirmed to have excellent efficacy in Experimental Example 2. Each anti-MSLN scFv and lentiviral vector were digested using Xhol (R0146S, NEB) and EcoRI (R0101, NEB at 37 °C for 2 hours, followed by agarose gel electrophoresis, and the identified products were purified using the FavorPrep Gel/PCR purification Mini kit (Favorgen). Each purified anti-MSLN scFv (100 ng) and vector (50 ng) were reacted at a ratio of 2:1 at 16 °C for 16 hours to perform ligation, and then transformed into Stbl3 competent cells to obtain colonies. The colonies were taken and grown in 5 mL of LB medium (ampicillin), and plasmid DNA was obtained using the DNA plasmid mini-prep method. It was confirmed that each anti-MSLN scFv inserted by cleaving the plasmid DNA with Xhol and EcoRI was well cloned into the vector. Afterwards, sequencing was performed to finally confirm the DNA sequence.

To the anti-MSLN scFv, CD8 hinge and CD8 transmembrane (TM) which are transmembrane regions, the cytoplasmic region of 4-1BB which is a signaling domain, and the intracellular domain of CD3 zeta (CD3z) which is a T cell activation domain were sequentially linked to construct anti-MSLN-CAR. In some embodiments, the anti-MSLN-CAR may include CD8 signal sequence (Signal peptide, SP) (SEQ ID NO: 51), MSLN34 scFv and its mutants (one selected from SEQ ID NO: 52 to 55), CD8 hinge region (SEQ ID NO: 56), CD8 transmembrane region (SEQ ID NO: 57), 4-1BB signaling domain (SEQ ID NO: 58) and CD3 zeta signaling domain (SEQ ID NO: 59). Each of the domains was linked sequentially using a corresponding restriction enzyme. Specific base sequence information corresponding to each domain is summarized in the table below.

[Table 15]

| Name | Nucleotide sequence (5'-3') | SEQ ID No. |
|---|---|---|
| CD8 | ATGGCCTTACCAGTGACCGCCTTGCTCCTGCCGCTGGCCTTG CTGCTCCACGCCGCCAGGCCG | 51 |
| MSLN34 scFv | GAAGTACAGTTGGTCGAAAGTGGCGGTGGCCTCGTGCAACC GGGTGGTTCACTGCGTCTGAGCTGCGCCGCCTCGGGTTTTAC TTTCTCTGATTATGGTATGCACTGGGTTCGTCAGGCGCCGGG CAAGGGTCTCGAATGGGTTTCATCTATCTACGGTTCTGGTGGT CACACTGGTTATGCCGATTCAGTGAAGGGTCGCTTTACCATTT CCCGTGACAACTCTAAGAATACTCTGTATCTGCAGATGAACTC GCTGCGTGCCGAAGACACGGCCGTCTATTATTGCGCCAAACA GCATGCATACCGTTACTCTTACGCATTCGATGTTTGGGGTCAG GGCACTTTAGTGACCGTCTCATCGGGTGGAGGCGGTTCAGG CGGAGGTGGATCCGGCGGTGGCGGATCGGACATTCAAATGA CGCAGAGTCCCTCCTCACTGAGTGCTAGCGTGGGCGATCGT GTGACAATTACTTGTCGCGCTAGCCAGTCTATCTCTAATTGGC TGAACTGGTATCAGCAGAAACCGGGCAAGGCGCCAAAATTGC TGATTTACGCAACTTCCTCTCTGCAGTCTGGTGTACCGTCCCG TTTCTCTGGCAGCGGTTCTGGTACGGATTTTACCCTGACCATC TCAAGCCTCCAGCCTGAAGATTTTGCCACCTATTATTGTCAGC AATCTTACTCTTTTCCGTTTACGTTCGGGCAGGGAACTAAAGT GGAAATTAAA | 52 |

(continued)

| Name | Nucleotide sequence (5'-3') | SEQ ID No. |
|---|---|---|
| MSLN34 D31L scFv | GAAGTACAGTTGGTCGAAAGTGGCGGTGGCCTCGTGCAACC GGGTGGTTCACTGCGTCTGAGCTGCGCCGCCTCGGGTTTTAC TTTCTCTCTTTATGGTATGCACTGGGTTCGTCAGGCGCCGGG CAAGGGTCTCGAATGGGTTTCATCTATCTACGGTTCTGGTGGT CACACTGGTTATGCCGATTCAGTGAAGGGTCGCTTTACCATTT CCCGTGACAACTCTAAGAATACTCTGTATCTGCAGATGAACTC GCTGCGTGCCGAAGACACGGCCGTCTATTATTGCGCCAAACA GCATGCATACCGTTACTCTTACGCATTCGATGTTTGGGGTCAG GGCACTTTAGTGACCGTCTCATCGGGTGGAGGCGGTTCAGG CGGAGGTGGATCCGGCGGTGGCGGATCGGACATTCAAATGA CGCAGAGTCCCTCCTCACTGAGTGCTAGCGTGGGCGATCGT GTGACAATTACTTGTCGCGCTAGCCAGTCTATCTCTAATTGGC TGAACTGGTATCAGCAGAAACCGGGCAAGGCGCCAAAATTGC TGATTTACGCAACTTCCTCTCTGCAGTCTGGTGTACCGTCCCG TTTCTCTGGCAGCGGTTCTGGTACGGATTTTACCCTGACCATC TCAAGCCTCCAGCCTGAAGATTTTGCCACCTATTATTGTCAGC AATCTTACTCTTTTCCGTTTACGTTCGGGCAGGGAACTAAAGT GGAAATTAAA | 53 |
| MSLN34 | GAAGTACAGTTGGTCGAAAGTGGCGGTGGCCTCGTGCAACC | 54 |

(continued)

| Name | Nucleotide sequence (5'-3') | SEQ ID No. |
|---|---|---|
| S192R scFv | GGGTGGTTCACTGCGTCTGAGCTGCGCCGCCTCGGGTTTTAC TTTCTCTGATTATGGTATGCACTGGGTTCGTCAGGCGCCGGG CAAGGGTCTCGAATGGGTTTCATCTATCTACGGTTCTGGTGGT CACACTGGTTATGCCGATTCAGTGAAGGGTCGCTTTACCATTT CCCGTGACAACTCTAAGAATACTCTGTATCTGCAGATGAACTC GCTGCGTGCCGAAGACACGGCCGTCTATTATTGCGCCAAACA GCATGCATACCGTTACTCTTACGCATTCGATGTTTGGGGTCAG GGCACTTTAGTGACCGTCTCATCGGGTGGAGGCGGTTCAGG CGGAGGTGGATCCGGCGGTGGCGGATCGGACATTCAAATGA CGCAGAGTCCCTCCTCACTGAGTGCTAGCGTGGGCGATCGT GTGACAATTACTTGTCGCGCTAGCCAGTCTATCTCTAATTGGC TGAACTGGTATCAGCAGAAACCGGGCAAGGCGCCAAAATTGC TGATTTACGCAACTTCCTCTCTGCAGCGTGGTGTACCGTCCC GTTTCTCTGGCAGCGGTTCTGGTACGGATTTTACCCTGACCAT CTCAAGCCTCCAGCCTGAAGATTTTGCCACCTATTATTGTCAG CAATCTTACTCTTTTCCGTTTACGTTCGGGCAGGGAACTAAAG TGGAAATTAAA | |

(continued)

| Name | Nucleotide sequence (5'-3') | SEQ ID No. |
|---|---|---|
| MSLN34 D31L/S1 92R scFv | GAAGTACAGTTGGTCGAAAGTGGCGGTGGCCTCGTGCAACC GGGTGGTTCACTGCGTCTGAGCTGCGCCGCCTCGGGTTTTAC TTTCTCTCTTTATGGTATGCACTGGGTTCGTCAGGCGCCGGG CAAGGGTCTCGAATGGGTTTCATCTATCTACGGTTCTGGTGGT CACACTGGTTATGCCGATTCAGTGAAGGGTCGCTTTACCATTT CCCGTGACAACTCTAAGAATACTCTGTATCTGCAGATGAACTC GCTGCGTGCCGAAGACACGGCCGTCTATTATTGCGCCAAACA GCATGCATACCGTTACTCTTACGCATTCGATGTTTGGGGTCAG GGCACTTTAGTGACCGTCTCATCGGGTGGAGGCGGTTCAGG CGGAGGTGGATCCGGCGGTGGCGGATCGGACATTCAAATGA CGCAGAGTCCCTCCTCACTGAGTGCTAGCGTGGGCGATCGT GTGACAATTACTTGTCGCGCTAGCCAGTCTATCTCTAATTGGC TGAACTGGTATCAGCAGAAACCGGGCAAGGCGCCAAAATTGC TGATTTACGCAACTTCCTCTCTGCAGCGTGGTGTACCGTCCC GTTTCTCTGGCAGCGGTTCTGGTACGGATTTTACCCTGACCAT CTCAAGCCTCCAGCCTGAAGATTTTGCCACCTATTATTGTCAG CAATCTTACTCTTTTCCGTTTACGTTCGGGCAGGGAACTAAAG TGGAAATTAAA | 55 |
| CD8 hinge | ACCACGACGCCAGCGCCGCGACCACCAACACCGGCGCCCAC CATCGCGTCGCAGCCCCTGTCCCTGCGCCCAGAGGCGTGCC GGCCAGCGGCGGGGGGCGCAGTGCACACGAGGGGGCTGGA CTTCGCCTGTGAT | 56 |
| CD8 TM | ATCTACATCTGGGCGCCCTTGGCCGGGACTTGTGGGGTCCTT CTCCTGTCACTGGTTATCACCCTTTACTGC | 57 |
| 4-1BB | AAACGGGGCAGAAAGAAACTCCTGTATATATTCAAACAACCAT TTATGAGACCAGTACAAACTACTCAAGAGGAAGATGGCTGTA GCTGCCGATTTCCAGAAGAAGAAGAAGGAGGATGTGAACTG | 58 |

(continued)

| Name | Nucleotide sequence (5'-3') | SEQ ID No. |
|---|---|---|
| CD3z | AGAGTGAAGTTCAGCAGGAGCGCAGACGCCCCCGCGTACAC AGCAGGGCCAGAACCAGCTCTATAACGAGCTCAATCTAGGAC GAAGAGAGGAGTACGATGTTTTGGACAAGAGACGTGGCCGG GACCCTGAGATGGGGGGAAAGCCGAGAAGGAAGAACCCTCA GGAAGGCCTGTACAATGAACTGCAGAAAGATAAGATGGCGGA GGCCTACAGTGAGATTGGGATGAAAGGCGAGCGCCGGAGGG GCAAGGGGCACGATGGCCTTTACCAGGGTCTCAGTACAGCCA CCAAGGACACCTACGACGCCCTTCACATGCAGGCCCTGCCCC CTCGC | 59 |

Cloning was completed, and the amino acid sequences of the scFv of the anti-MSLN chimeric antigen receptor vector, with increased and improved affinity, including WT, single mutants of D31L, and S192R and mutation comprised mutant D31L and mutant S192R of MSLN 34, was identified.

**3.2 Production of lentivirus loaded with anti-MSLN-CAR and measurement of functional titer**

The results of confirming the functional titer of the CAR-loaded lentivirus targeting HeLa cells are shown in Table 16 [Infectious titer measurement results (FACS analysis)].

[Table 16]

| Sample | Titer measured (TU/mL) |
|---|---|
| MSLN34 | $1.76 \times 10^7$ |
| MSLN34-D31L | $1.74 \times 10^7$ |
| MSLN34-S192R | $2.20 \times 10^7$ |
| MSLN34-D31L/S192R | $1.65 \times 10^7$ |

**3.3 Construction of cells into which anti-MSLN-CAR was introduced**

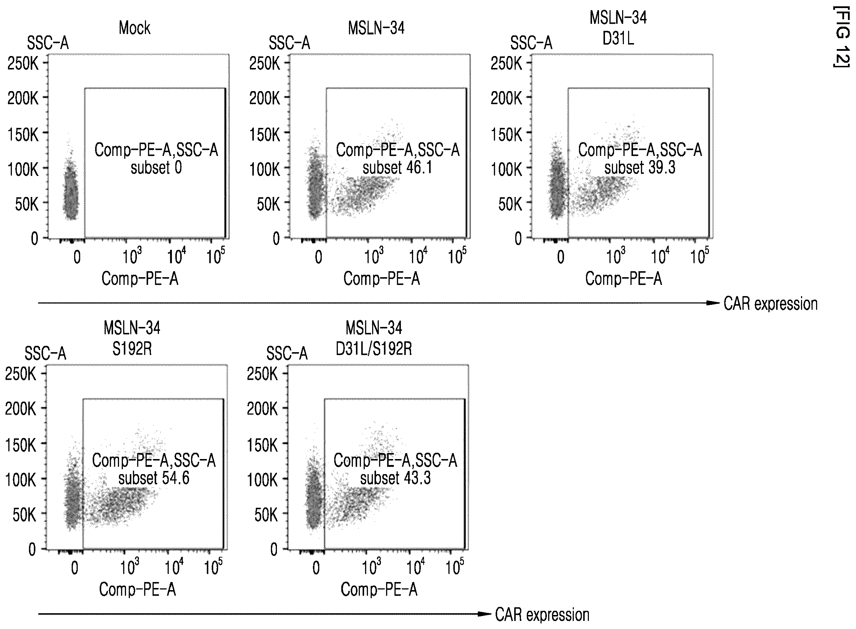

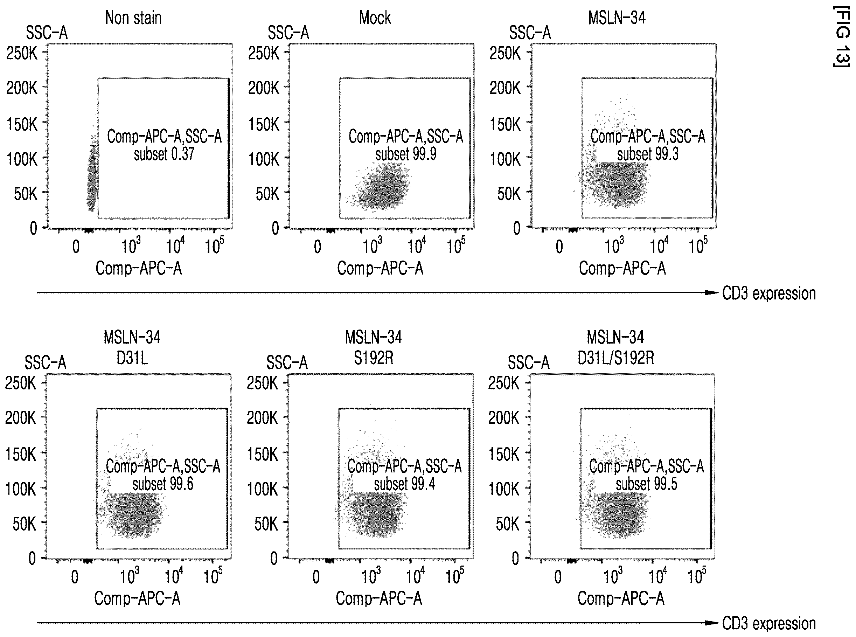

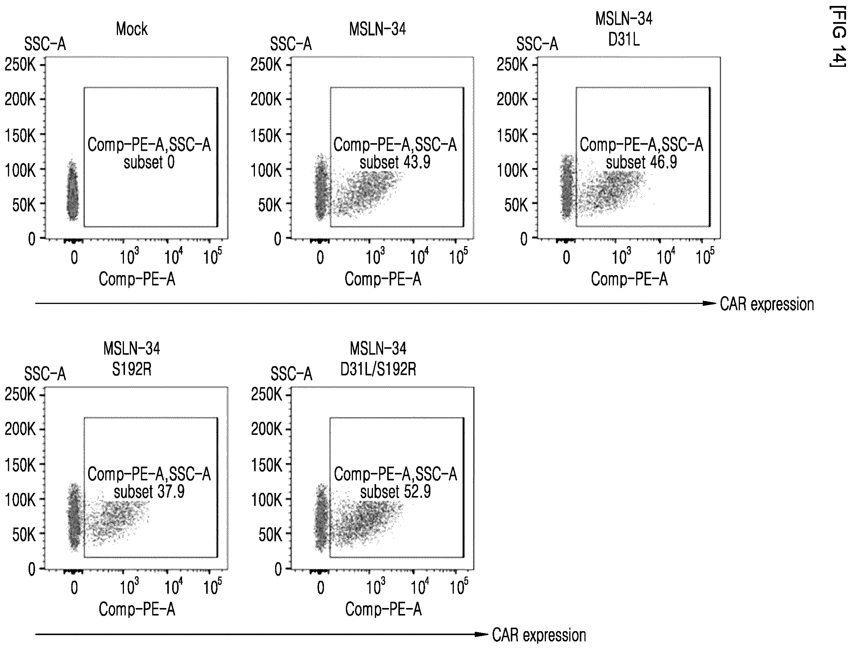

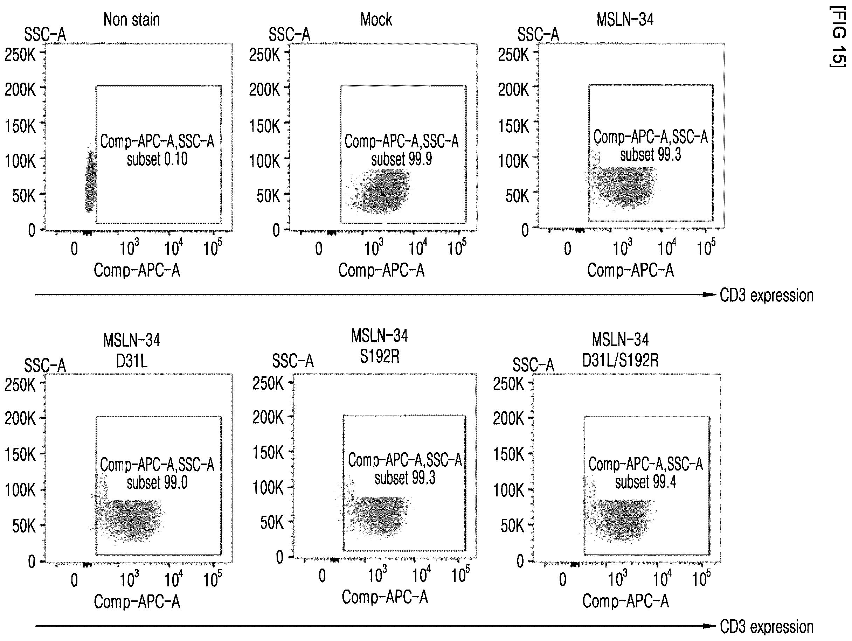

An experiment (Batch #1 and Batch #2) was performed to produce MSLN CAR-T cells into which the vector of Experimental Example 3.1 was introduced, and characteristics thereof were analyzed through FACS analysis. The results obtained by analyzing the characteristics of the MSLN CAR-T produced in Batch #1 are shown in FIGS. 12 and 13, and the results obtained by analyzing the characteristics of the MSLN CAR-T in the produced Batch #2 are shown in FIGS. 14 and 15. Additionally, the characteristics analysis results of Batch #1 and #2 MSLN CAR-T are summarized in Table 17.

As confirmed in FIGS. 12 and 13, CAR-expressing cells in MSLN CAR-T Batch #1 were 46.1%, 39.3%, 54.6%, and 43.3% in MSLN34, MSLN34-D31L, MSLN34-S192R, and MSLN34-D31L/S192R, respectively. Most CAR-expressing cells were identified as $CD3^+$ T cells ($\geq$ 99.3%).

Additionally, as confirmed in FIGS. 14 and 15, CAR expression cells in MSLN CAR-T Batch #2, were 43.9%, 46.9%, 37.9%, 52.9% in MSLN34, MSLN34-D31L, MSLN34-S192R, and MSLN34-D31L/S192R, respectively. Most CAR-expressing cells were identified as $CD3^+$ T cells ($\geq$ 99.0%).

[Table 17]

| CAR-T Production Batch # | Group | CAR expression (%) | CD3 |
|---|---|---|---|
| **Batch #1** | Mock T cell | < 1 | ≥ 99.9% |
| | MSLN34 CAR-T cell | 46.1 | ≥ 99.3% |
| | MSLN34-D31L CAR-T cell | 39.3 | ≥ 99.6% |
| | MSLN34-S192R CAR-T cell | 54.6 | ≥ 99.4% |
| | MSLN34-D31L/S192R CAR-T cell | 43.3 | ≥ 99.5% |
| **Batch #2** | Mock T cell | < 1 | ≥ 99.9% |
| | MSLN34 CAR-T cell | 43.9 | ≥ 99.3% |
| | MSLN34-D31L CAR-T cell | 46.9 | ≥ 99.0% |
| | MSLN34-S192R CAR-T cell | 37.9 | ≥ 99.3% |
| | MSLN34-D31L/S192R CAR-T cell | 52.9 | ≥ 99.4% |

## Experimental Example 4: Confirmation of cytotoxic effect of anti-MSLN-CAR-T cells with increased and improved affinity on mesothelioma and ovarian cancer

Using the anti-MSLN-CAR-T cells prepared in Experimental Example 3, the cytotoxic effect on mesothelioma and ovarian cancer cells was confirmed through Calcein-AM (calcein release assay).

First, the *in vitro* efficacy of CAR-T on NCI-H2052, which is a malignant pleural mesothelioma cell line, and OVCAR-3, which is an ovarian cancer cell line, was evaluated through batch #1 and batch #2 experiments for cytotoxic effects on target cells using Calcein-AM. The statistical significance of the evaluation results was determined using two-way ANOVA (Full model, Tukey, 95% confidence interval) (ns, $P > 0.05$; *, $P \leq 0.05$; **, $P \leq 0.01$; ***, $P \leq 0.001$ vs. the Mock). In addition, the results of an experiment on the cytotoxic effect of anti-MSLN-CAR-T cells using Calcein-AM on target cells are shown in Table 18.

[Table 18]

| CAR-T Production Batch # | Target cell | E:T | % of specific lysis | | | | |
|---|---|---|---|---|---|---|---|
| | | | Mock | MSLN34 | MSLN34 D31L | MSLN34 S192R | MSLN34 D31L/S1 92R |
| #1 | NCI-H2052 | 10:1 | -0.2 | 51.9 | 76.6 | 41.2 | 57.9 |
| | | 5:1 | 1.9 | 30.3 | 69.7 | 28.2 | 45.5 |
| | | 2.5:1 | 0.3 | 21.7 | 55.7 | 21.5 | 36.5 |
| | | 1.25:1 | 1.7 | 12.2 | 40.1 | 8.8 | 24.4 |
| | OVCAR-3 | 10:1 | -1.9 | 36.1 | 55.4 | 25.6 | 45.4 |
| | | 5:1 | 0.5 | 25.9 | 54.6 | 27.1 | 41.1 |
| | | 2.5:1 | 3.1 | 13.9 | 29.4 | 14.3 | 26.1 |
| | | 1.25:1 | 0.5 | 9.6 | 22.2 | 9.8 | 19.9 |
| #2 | NCI-H2052 | 10:1 | 3.7 | 54.8 | 71.5 | 50.4 | 62.3 |
| | | 5:1 | 1.7 | 37.3 | 53.4 | 35.7 | 56.2 |
| | | 2.5:1 | 4.5 | 29.3 | 59.9 | 32.4 | 53.1 |
| | | 1.25:1 | 1.8 | 20.0 | 44.4 | 22.0 | 45.1 |
| | OVCAR | 10:1 | -0.1 | 25.1 | 50.4 | 26.1 | 44.6 |
| | | 5:1 | 0.3 | 17.9 | 41.1 | 18.6 | 38.6 |
| | | 2.5:1 | 1.4 | 13.2 | 30.1 | 14.5 | 32.3 |
| | | 1.25:1 | 0.5 | 9.2 | 21.8 | 8.6 | 21.1 |

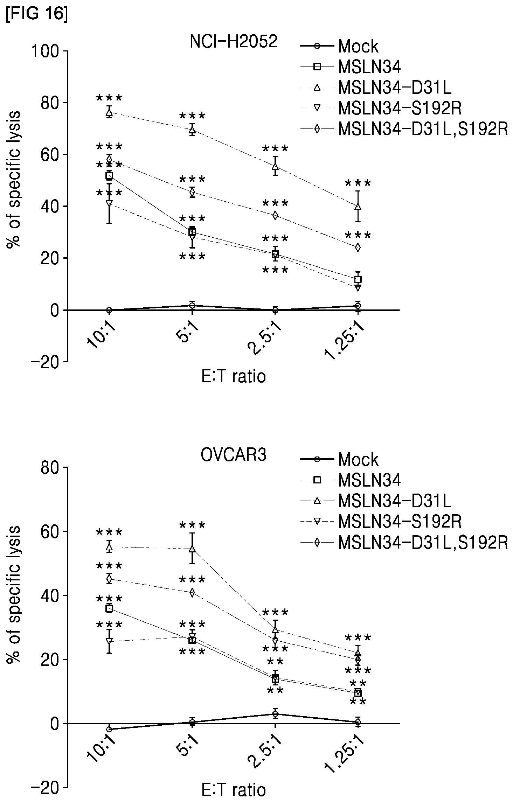

The results of the first Calcein-AM using MSLN CAR-T batch #1 are shown in FIG. 16. As confirmed in FIG. 11, the cytotoxic effects of MSLN34-D31L CAR-T and MSLN34-D31L/S192R CAR-T on the NCI-H2052 target cell line were increased by 24.7% and 6.0 %, respectively, and the cytotoxic effect of MSLN34-S192R CAR-T was reduced by 10.7 %, compared to MSLN34 CAR-T cells based on the E:T = 10:1 experimental group. The cytotoxic effects of MSLN34-D31L CAR-T and MSLN34-D31L/S192R CAR-T on the OVCAR-3 target cell line were increased by 19.3% and 9.3%, respectively, and the cytotoxic effect of MSLN34-S192R CAR-T was reduced by 10.5%, compared to MSLN34 CAR-T cells based on the E:T = 10:1 experimental group.

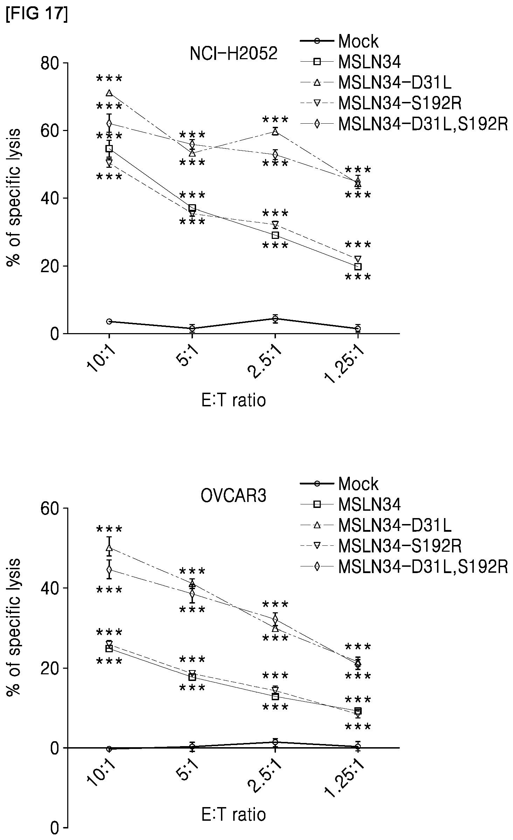

The results of the second Calcein-AM using MSLN CAR-T batch #2 are shown in FIG. 17. As confirmed in FIG. 17, the cytotoxic effects of MSLN34-D31L CAR-T and MSLN34-D31L/S192R CAR-T on the NCI-H2052 target cell line were increased by 16.7% and 7.5%, respectively, and the cytotoxic effect of MSLN34-S192R CAR-T was reduced by 4.4 %, compared to MSLN34 CAR-T cells based on the E:T = 10:1 experimental group. The cytotoxic effects of MSLN34-D31L CAR-T and MSLN34-D31L/S192R CAR-T on the OVCAR-3 target cell line were increased by 25.3% and 19.5%, respectively, and the cytotoxic effect of MSLN34-S192R CAR-T was reduced by 1.0%, compared to MSLN34 CAR-T cells based on the E:T = 10:1 experimental group.

Therefore, it was confirmed that in mesothelioma and ovarian cancer cell lines, MSLN34-D31L and MSLN34-D31L/S192R showed higher cancer cell cytotoxic ability compared to MSLN34 CAR-T.

**Experimental Example 5: Confirmation of the cytotoxic effect of anti-MSLN-CAR-T cells with increased and improved affinity on pancreatic cancer**

The cytotoxic effect on pancreatic cancer cells was confirmed using the anti-MSLN-CAR-T cells prepared in Experimental Example 3 through an Incucyte-based real-time cytotoxicity assay.

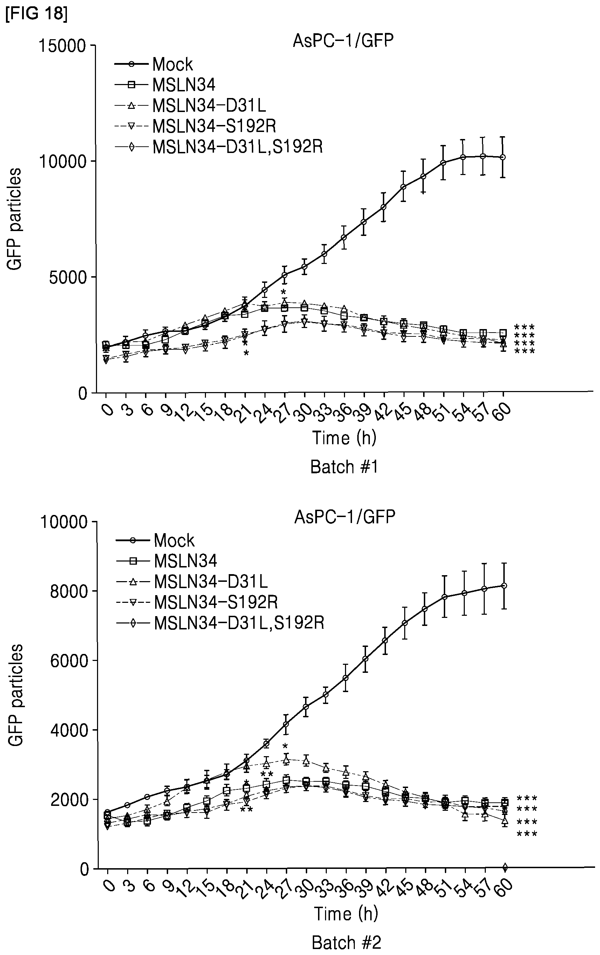

Regarding the *in vitro* cancer cell cytotoxic effect of anti-MSLN-CAR-T cells with increased and improved affinity, GFP particles were analyzed using incucyte to evaluate the cytotoxic effect on target cells (AsPC-1/GFP, pancreatic cancer cells). Results thereof are shown in FIG. 18. The statistical significance of the evaluation results was determined using two-way ANOVA (Full model, Tukey, 95% confidence interval) (ns, $P > 0.05$; *, $P \leq 0.05$; **, $P \leq 0.01$; ***, $P \leq 0.001$ vs. the Mock).

As confirmed in FIG. 18, as a result of the first and second experiments using MSLN CAR-T batch #1 & batch #2, compared to mock T cell at E:T ratio 0.5, MSLN34-D31L, MSLN34-S192R, MSLN34-D31L/S192R CAR-T cells all exhibited a statistically significant cytotoxic effect on pancreatic cancer cells, with GFP particles changing from an increasing trend to a decreasing trend from 21 h to 27 h.

**Experimental Example 6: Confirmation of anticancer efficacy of anti-MSLN-CAR-T cells with increased and improved affinity in animal model**

**6.1 Observation of body weight changes and general symptoms**

Body weight changes and general symptoms were identified in pancreatic cancer animal models treated with anti-MSLN-CAR-T cells.

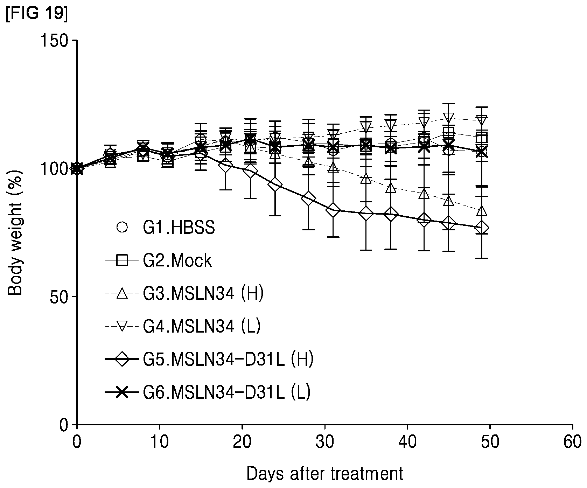

As a result, a total of 1 animal (G5-2) died during the test period, and body weight was decreased in some groups. Body weight loss was observed in the group administered at high concentration ($1.5 \times 10^6$ cells/head), and some deaths occurred. Specifically, compared to the vehicle control group (G1), the MSLN34 (WT) high concentration (G3) group showed the decrease in the body weight starting from day 38 after administration, and the MSLN34-D31L high concentration (G5) group showed the decrease in the body weight starting from day 24 after administration ($p<0.05$). In addition, compared to the mock administration group (G2), the MSLN34 (WT) high concentration (G3) group showed the decrease in the body weight starting from day 42 after administration, and the MSLN34-D31L high concentration (G5) group showed the decrease in the body weight starting from day 24 after administration ($p<0.05$) (FIG. 19, table 19, and table 20).

Table 19 below shows the mortality rate of pancreatic cancer animal models after CAR-T cell treatment, expressed as number of dead subjects/total number of subjects. Dead subjects appeared on day 42 after CAR-T cell treatment.

[Table 19]

| Days | G1 | G2 | G3 | G4 | G5 | G6 |
|------|-----|-----|-----|-----|-----|-----|
| 0 | 0/5 | 0/5 | 0/5 | 0/5 | 0/5 | 0/5 |
| 42 | 0/5 | 0/5 | 0/5 | 0/5 | 0/5 | 0/5 |

(continued)

| Days | G1 | G2 | G3 | G4 | G5 | G6 |
|------|-----|-----|-----|-----|-----|-----|
| 45 | 0/5 | 0/5 | 0/5 | 0/5 | 0/5 | 0/5 |
| 49 | 0/5 | 0/5 | 0/5 | 0/5 | 1/5 | 0/5 |

FIG. 19 and table 20 show changes in body weight of pancreatic cancer animal models after CAR-T cell treatment, with each value expressed as the mean ± standard deviation of body weight (%). The administration day was designated as day 0, and the measured body weight was expressed as a percentage (%) of body weight divided by the body weight on day 0. The data also includes data of subjects who died during the experiment. Statistical analysis was performed using one-way ANOVA and tukey's post hoc test (*: p<0.05, **: p<0.01, ***: p<0.001 vs vehicle control group (G1), #: p<0.05, ##: p<0.01, ###: p<0.001 vs mock cell treatment group (G2)).

[Table 20]

| Day s | G1 | G2 | G3 | G4 | G5 | G6 |
|-------|-----|-----|-----|-----|-----|-----|
| 0 | 100.0 ± 0.0 | 100.0 ± 0.0 | 100.0 ± 0.0 | 100.0 ± 0.0 | 100.0 ± 0.0 | 100.0 ± 0.0 |
| 4 | 103.4 ± 0.7 | 103.8 ± 0.6 | 102.7 ± 1.9 | 105.6 ± 3.5 | 105.3 ± 1.7 | 104.2 ± 2.4 |
| 8 | 107.0 ± 2.8 | 105.0 ± 1.6 | 106.9 ± 1.5 | 105.7 ± 2.5 | 107.4 ± 2.4 | 108.2 ± 2.8 |
| 11 | 105.5 ± 4.8 | 102.7 ± 1.7 | 104.6 ± 2.8 | 106.0 ± 3.6 | 104.8 ± 1.1 | 105.9 ± 4.0 |
| 15 | 111.3 ± 6.1 | 106.3 ± 1.8 | 109.0 ± 5.8 | 108.4 ± 5.0 | 105.8 ± 6.6 | 108.2 ± 6.3 |
| 18 | 110.5 ± 5.2 | 108.3 ± 3.1 | 111.3 ± 3.3 | 111.7 ± 4.2 | 101.2 ± 9.4 | 109.0 ± 5.9 |
| 21 | 110.9 ± 8.2 | 108.5 ± 5.0 | 108.0 ± 6.2 | 110.8 ± 4.4 | 99.1 ± 10.8 | 111.7 ± 6.0 |
| 24 | 112.2 ± 6.4 | 107.9 ± 4.4 | 105.8 ± 3.8 | 111.7 ± 4.8 | 93.8 ± 12.3***,# | 108.4 ± 6.4 |
| 28 | 110.2 ± 8.9 | 109.8 ± 3.8 | 102.6 ± 5.1 | 112.1 ± 5.2 | 88.5 ± 12.4***,## | 109.0 ± 4.4 |
| 31 | 107.2 ± 8.0 | 109.6 ± 3.2 | 100.6 ± 7.5 | 112.8 ± 4.5 | 83.9 ± 10.7***,### | 108.6 ± 4.4 |
| 35 | 109.6 ± 8.9 | 108.8 ± 3.1 | 96.2 ± 9.1 | 115.8 ± 4.3 | 82.5 ± 14.3***,## | 109.0 ± 5.2 |
| 38 | 109.8 ± 9.0 | 108.5 ± 0.5 | 92.7 ± 10.4* | 116.7 ± 4.3 | **82.2** ± 13.6***,## | 107.9 ± 6.8 |
| 42 | 112.1 ± 10.9 | 110.6 ± 2.3 | 90.4 ± 11.2**,# | 117.8 ± 3.5 | 80.2 ± 12.3***,### | 108.7 ± 4.7 |
| 45 | 107.2 ± 9.4 | 114.0 ± 2.8 | 87.4 ± 11.2**,## | 119.6 ± 5.6 | **78.9** ± 11.2***,### | 109.1 ± 3.8 |
| 49 | 106.5 ± 13.4 | 112.2 ± 2.9 | 83.8 ± 9.2**,### | 118.5 ± 5.5 | 77.0 ± 12.0***,### | 106.7 ± 3.6 |

## 6.2 Tumor growth/reduction assessment

The level of tumor growth/reduction was evaluated in a pancreatic cancer animal model treated with anti-MSLN-CAR-T cells.

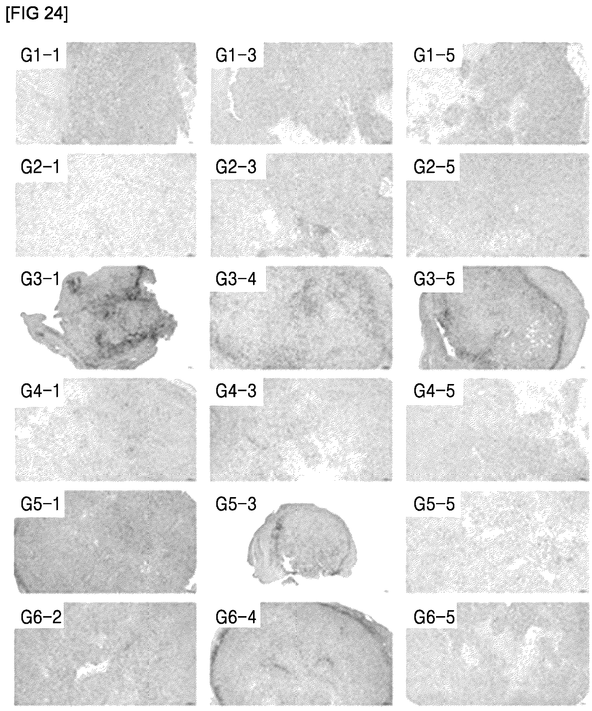

As a result, compared to the vehicle control group (G1), the mock administration group (G2) had a statistically significant tumor decrease starting from day 35 after administration (p<0.01 vs. G1). In addition, compared to the vehicle control group (G1), tumors were statistically significantly reduced in all MSLN-CAR-T administration groups (G3 to G10) (p<0.05 vs. G1) (FIG. 24 and table 19).

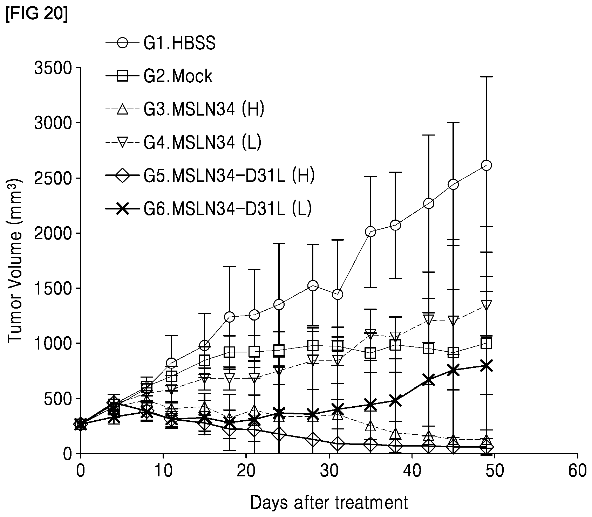

FIG. 20 and table 21 show changes in tumor volume of pancreatic cancer animal models after CAR-T cell treatment, with each value expressed as the mean ± standard deviation of tumor volume (mm$^3$). In the case where there was no detectable tumor, it was marked as '0'. The data also includes data of subjects who died during the experiment. Statistical analysis was performed using one-way ANOVA and tukey's post hoc test (*: p<0.05, **: p<0.01, ***: p<0.001 vs vehicle control group (G1), #: p<0.05, ##: p<0.01, ###: p<0.001 vs mock cell treatment group (G2)).

[Table 21]

| Days | G1 | G2 | G3 | G4 | G5 | G6 |
|------|-----|-----|-----|-----|-----|-----|
| 0 | 275.7 ± 48.8 | 276.8 ± 46.0 | 277.5 ± 44.9 | 276.3 ± 46.2 | 275.7 ± 50.2 | 275.3 ± 51.9 |
| 4 | 437.4 ± 101.4 | 454.8 ± 81.6 | 435.7 ± 67.0 | 459.5 ± 56.3 | 471.0 ± 77.9 | 343.4 ± 63.3 |

(continued)

| Days | G1 | G2 | G3 | G4 | G5 | G6 |
|------|----|----|----|----|----|----|
| 8 | 599.1 ± 108.8 | 617.3 ± 84.9 | 498.1 ± 89.5 | 560.2 ± 85.1 | 400.4 ± 79.2[*,#] | 390.4 ± 79.8[**,#] |
| 11 | 827.9 ± 249.6 | 711.3 ± 85.3 | 419.9 ± 61.8[***,#] | 595.4 ± 125.7 | 326.2 ± 72.7[***,##] | 328.1 ± 88.8[***,##] |
| 15 | 987.1 ± 291.8 | 853.8 ± 120.5 | 436.8 ± 120.4[***,##] | 688.0 ± 94.5[*] | 289.1 ± 71.2[***,###] | 337.9 ± 153.1[***,###] |
| 18 | 1247.2 ± 459.1 | 928.1 ± 148.9 | 341.6 ± 67.1[***,##] | 691.5 ± 105.6[**] | 233.6 ± 78.6[***,###] | 294.6 ± 259.6[***,###] |
| 21 | 1264.4 ± 413.6 | 930.8 ± 144.7 | 405.7 ± 136.8[***,#] | 693.2 ± 152.4[**] | 230.6 ± 103.3[***,##] | 319.5 ± 372.7[***,##] |
| 24 | 1360.0 ± 552.3 | 949.5 ± 164.0 | 351.6 ± 60.4[***,#] | 758.1 ± 120.7[**] | **187.5 ± 47.1[***,##]** | 377.6 ± 523.8[***,#] |
| 28 | 1534.8 ± 364.6[#] | 987.7 ± 160.7 | 345.8 ± 45.6[***,#] | 854.5 ± 260.9[**] | 145.9 ± 34.8[***,###] | 372.2 ± 531.2[***,#] |
| 31 | 1450.0 ± 497.3 | 984.2 ± 172.6 | 368.4 ± 129.2[***] | 852.0 ± 208.8[*] | 106.2 ± 40.9[***,##] | 417.0 ± 617.7[***] |
| 35 | 2018.0 ± 498.9[###] | 921.3 ± 176.4[***] | 267.9 ± 132.2[***] | 1085.7 ± 232.9[**] | 97.4 ± 37.5[***,#] | 460.0 ± 643.7[***] |
| 38 | 2075.7 ± 481.0[##] | 994.9 ± 247.7[***] | 203.7 ± 100.2[***,#] | 1058.9 ± 194.6[**] | 81.9 ± 60.3[***,##] | 493.6 ± 751.1[***] |
| 42 | 2274.7 ± 621.4[##] | 961.2 ± 324.4[***] | 178.2 ± 83.2[***] | 1219.7 ± 197.7[**] | 78.7 ± 97.0[***] | 671.5 ± 984.3[***] |
| 45 | 2444.7 ± 558.3[##] | 922.3 ± 332.4[***] | 140.9 ± 36.8[***] | 1204.1 ± 297.9[**] | 72.3 ± 86.1 [***] | 763.7 ± 1187.3[***] |
| 49 | 2623.1 ± 795.3[##] | 1011.7 ± 467.4[**] | 143.8 ± 80.8[***] | 1344.5 ± 267.8[*] | 74.4 ± 73.3[***] | 807.2 ± 1257.5[***] |

### 6.2.1 MSLN34 (WT) treatment group

Compared to the vehicle control group (G1), tumors in the MSLN34 (WT) high concentration (G3) group were decreased starting from day 11 after administration (p<0.05 vs. G1), and tumors in the MSLN34 (WT) low concentration (G4) group were decreased starting from day 15 after administration (p<0.05 vs. G1).

Compared to the mock administration group (G2), statistical significance was verified in the MSLN34 (WT) high concentration (G3) group from day 11 to day 28, and day 38 after administration (p<0.05 vs. G2), and in the case of the MSLN34 (WT) low concentration (G4) group, statistical significance was not verified.

The MSLN34 (WT) high concentration (G3) group showed a tendency for tumors to decrease, but the MSLN34 (WT) low concentration (G4) group showed a tendency for tumors to increase.

### 6.2.2 MSLN34-D31L treatment group

Compared to the vehicle control group (G1), tumors in the MSLN34-D31L high concentration (G5) and low concentration (G6) groups were decreased starting from day 8 after administration (p<0.05 vs. G1).

Compared to the mock administration group (G2), in the case of the MSLN34-D31L high concentration (G5) group, statistical significance was verified from day 8 to day 38 after administration and in the case of the MSLN34-D31L low concentration (G6) group, statistical significance was verified from day 8 to day 28 after administration (p <0.05 vs. G1).

In the case of the MSLN34-D31L administration groups (G5, G6), an initial tendency for tumor reduction was observed regardless of the administration concentration. However, over time, one subject in the low concentration (G6) group showed a tendency for tumors to increase, and in the blood analysis of the corresponding subject, a low level of hCD3% was observed, suggesting that T cell proliferation did not occur in the body.

**6.3 Tumor weight measurement**

Tumor weight was measured/evaluated in a pancreatic cancer animal model treated with anti-MSLN-CAR-T cells.

As a result, tumor weight was decreased in all administration groups compared to the vehicle control group (G1) (p<0.01 vs. G1).

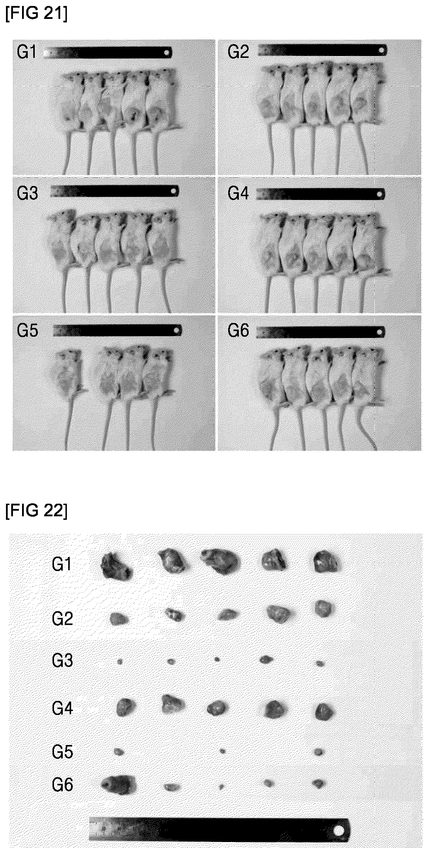

Compared to the mock administration group (G2), the MSLN34 (WT) high concentration (G3) group showed the tumor weight decrease of an average of 93%, and the MSLN34-D31L high concentration (G5) group showed the tumor weight decrease of an average of 91%. Additionally, in the MSLN34-D31L high concentration (G5) group, a subject in which the tumor was completely removed was found. However, compared to the mock administration group (G2), the average tumor weight in the MSLN34 (WT) low concentration (G4) group was increased, and the average tumor weight in the MSLN34-D31L low concentration (G6) group was decreased by 18%. In the MSLN34-D31L low concentration (G6) group, specifically, one subject showed an increase in tumors, resulting in a large standard deviation. In the blood analysis of the subject, a low level of hCD3% was observed, suggesting that T cell proliferation did not occur in the body (FIGS. 21 to 23 and table 22).

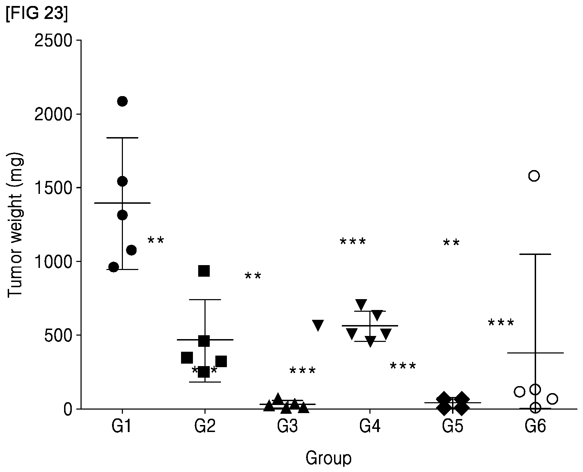

FIG. 23 and Table 22 show changes in weight of pancreatic cancer animal models after CAR-T cell treatment, with each value expressed as the mean $\pm$ standard deviation of weight (mg). The pancreatic cancer animal model was euthanized on day 49 after CAR-T cell treatment and the tumor weight was measured. When no tumor was found, it was expressed as Omg. Statistical analysis was performed using one-way ANOVA and tukey's post hoc test (*: p<0.05, **: p<0.01, ***: p<0.001 vs vehicle control group (G1), #: p<0.05, ##: p<0.01, ###: p<0.001 vs mock treatment group (G2)).

[Table 22]

|  | G1 | G2 | G3 | G4 | G5 | G6 |
|---|---|---|---|---|---|---|
| **(mg)** | 1394.6 $\pm$ | 462.8 $\pm$ | 30.8 $\pm$ | 560.0 $\pm$ | 39.3 $\pm$ | 378.5 $\pm$ |
|  | 447.3## | 276.1** | 23.7*** | 102.5** | 38.2*** | 671.6*** |

**6.4 Histopathological evaluation**

Histopathological levels were identified in a pancreatic cancer animal model treated with anti-MSLN-CAR-T cells. Specifically, 3 animals per group were selected and immunohistochemical staining (IHC) for hCD3ε was performed.

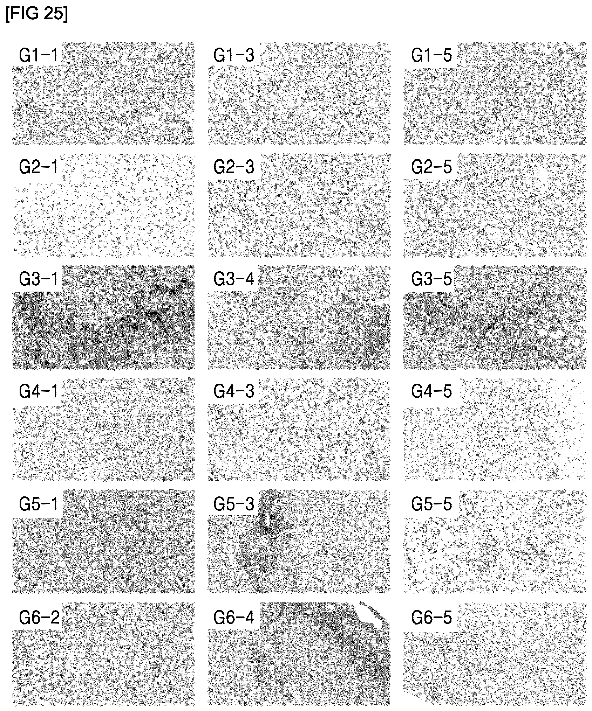

As a result, in the vehicle control group (G1), little T cell infiltration was observed within the tumor. In the mock administration group (G2), sporadic T cell infiltration was identified within the tumor. T cell infiltration within the tumor was found to be highest in the MSLN34 (WT) high concentration (G3) group. When the same type of CAR-T cells were administered, high T cell infiltration was confirmed at a relatively high concentration ($1.5 \times 10^6$ cells/head). When the same costimulatory factors were used, MSLN34-D31L with an optimized scFv region did not show higher T cell infiltration compared to the existing MSLN34 (FIGS. 24 and 25).

The description of the present disclosure described above is for illustrative purposes, and those skilled in the art would understand that the present disclosure could be easily modified into other specific forms without changing the technical concept or essential features of the present disclosure. Therefore, the embodiments described above should be understood in all respects as illustrative and not restrictive.

This research was supported by the Korea Drug Development Fund funded by the Ministry of Science and ICT, the Ministry of Trade, Industry, and Energy, and the Ministry of Health and Welfare (RS-2023-00217215, Republic of Korea).

**Claims**

1. An anti-mesothelin antibody or an antigen-binding fragment thereof, comprising:

   a heavy chain variable region including heavy chain complementarity determining region 1 (HCDR1) including an amino acid sequence consisting of SEQ ID NO: 19, heavy chain complementarity determining region 2 (HCDR2) including an amino acid sequence consisting of SEQ ID NO: 20, and a heavy chain complementarity determining region 3 (HCDR3) including an amino acid sequence consisting of SEQ ID NO: 21; and
   a light chain variable region including light chain complementarity determining region 1 (LCDR1) including an amino acid sequence consisting of SEQ ID NO: 22, light chain complementarity determining region 2 (LCDR2) including an amino acid sequence consisting of SEQ ID NO: 23, and light chain complementarity determining region 3 (LCDR3) including an amino acid sequence consisting of SEQ ID NO: 24,
wherein the heavy chain variable region and the light chain variable region include one or more amino acid substitutions.

2. The anti-mesothelin antibody or the antigen-binding fragment thereof of claim 1, wherein the one or more amino acid substitutions occur at one or more positions selected from the group consisting of a 1st position of SEQ ID NO: 19, a 7th position of SEQ ID NO: 23, and a 4th position of SEQ ID NO: 24.

3. The anti-mesothelin antibody or the antigen-binding fragment thereof of claim 1, wherein the one or more amino acid substitutions include one or more selected from the group consisting of the following amino acid substitutions:

   1) a 1st amino acid of SEQ ID NO: 19 is substituted from D to K, W, L or R,
   2) a 7th amino acid of SEQ ID NO: 23 is substituted from S to F or R; and
   3) a 4th amino acid of SEQ ID NO: 24 is substituted from Y to R.

4. The anti-mesothelin antibody or the antigen-binding fragment thereof of claim 1, wherein the one or more amino acid substitutions are selected from the group consisting of the following amino acid substitutions:

   1) a 1st amino acid of SEQ ID NO: 19 is substituted from D to L,
   2) a 7th amino acid of SEQ ID NO: 23 is substituted from S to R; and
   3) a 1st amino acid of SEQ ID NO: 19 is substituted from D to L, and a 7th amino acid of SEQ ID NO: 23 is substituted from S to R.

5. The anti-mesothelin antibody or the antigen-binding fragment thereof of claim 1, wherein the anti-mesothelin antibody or the antigen-binding fragment thereof is selected from antibodies or antigen-binding fragments thereof, including a heavy chain variable region including the following heavy chain CDRs and a light chain variable region including the following light chain CDRs:

   1) an antibody or an antigen-binding fragment thereof, including: a heavy chain variable region including HCDR1 including an amino acid sequence consisting of SEQ ID NO: 27, HCDR2 including an amino acid sequence consisting of SEQ ID NO: 28, and HCDR3 including an amino acid sequence consisting of SEQ ID NO: 29; and a light chain variable region including LCDR1 including an amino acid sequence consisting of SEQ ID NO: 30, LCDR2 including an amino acid sequence consisting of SEQ ID NO: 31, and LCDR3 including an amino acid sequence consisting of SEQ ID NO: 32,
   2) an antibody or an antigen-binding fragment thereof, including: a heavy chain variable region including HCDR1 including an amino acid sequence consisting of SEQ ID NO: 35, HCDR2 including an amino acid sequence consisting of SEQ ID NO: 36, and HCDR3 including an amino acid sequence consisting of SEQ ID NO: 37; and a light chain variable region including LCDR1 including an amino acid sequence consisting of SEQ ID NO: 38, LCDR2 including an amino acid sequence consisting of SEQ ID NO: 39, and LCDR3 including an amino acid sequence consisting of SEQ ID NO: 40, and
   3) an antibody or an antigen-binding fragment thereof, including: a heavy chain variable region including HCDR1 including an amino acid sequence consisting of SEQ ID NO: 43, HCDR2 including an amino acid sequence consisting of SEQ ID NO: 44, and HCDR3 including an amino acid sequence consisting of SEQ ID NO: 45; and a light chain variable region including LCDR1 including an amino acid sequence consisting of SEQ ID NO: 46, LCDR2 including an amino acid sequence consisting of SEQ ID NO: 47, and LCDR3 including an amino acid sequence consisting of SEQ ID NO: 48.

6. The anti-mesothelin antibody and the antigen-binding fragment thereof of claim 1, wherein the anti-mesothelin antibody or the antigen-binding fragment thereof is selected from antibodies or antigen-binding fragments thereof including the following heavy chain variable regions and the following light chain variable regions:

   1) an antibody or an antigen-binding fragment thereof including a heavy chain variable region including an amino acid sequence consisting of SEQ ID NO: 33 and a light chain variable region including an amino acid sequence consisting of SEQ ID NO: 34;
   2) an antibody or an antigen-binding fragment thereof including a heavy chain variable region including an amino acid sequence consisting of SEQ ID NO: 41 and a light chain variable region including an amino acid sequence consisting of SEQ ID NO: 42; and
   3) an antibody or an antigen-binding fragment thereof including a heavy chain variable region including an amino acid sequence consisting of SEQ ID NO: 49 and a light chain variable region including an amino acid sequence consisting of SEQ ID NO: 50.

7. The anti-mesothelin antibody and the antigen-binding fragment thereof of claim 1, wherein the anti-mesothelin antibody or the antigen-binding fragment thereof is selected from antibodies or antigen-binding fragments thereof including the following antigen-binding fragments:

   1) an antibody or an antigen-binding fragment thereof, including an antigen-binding fragment that includes the amino acid sequence consisting of SEQ ID NO: 2;
   2) an antibody or antigen-binding fragment thereof, including an antigen-binding fragment that includes the amino acid sequence consisting of SEQ ID NO: 3; and
   3) an antibody or an antigen-binding fragment thereof, including an antigen-binding fragment that includes the amino acid sequence consisting of SEQ ID NO: 4.

8. The anti-mesothelin antibody and the antigen-binding fragment thereof of claim 1, wherein the one or more amino acid substitutions increase the affinity of the anti-mesothelin antibody or the antigen-binding fragment thereof with respect to mesothelin.

9. An isolated nucleic acid encoding the antibody or the antigen-binding fragment thereof according to any one of claims 1 to 8.

10. A vector including the isolated nucleic acid of claim 9.

11. An isolated host cell transformed with the vector of claim 10.

12. A method of producing an anti-mesothelin antibody comprising culturing the isolated host cell of claim 11 to express an antibody.

13. A chimeric antigen receptor comprising an antigen-binding domain, a hinge domain, a transmembrane domain, and an intracellular signaling domain, wherein the antigen-binding domain is a chimeric antigen receptor including the antibody or the antigen-binding fragment thereof according to one of claims 1 to 8.

14. The chimeric antigen receptor of claim 13, wherein the antigen-binding fragment is a single chain variable fragment (scFv).

15. A polynucleotide encoding the chimeric antigen receptor of claim 13.

16. The polynucleotide of claim 15, wherein the polynucleotide comprises one or more base sequences selected from the group consisting of SEQ ID NOS: 53 to 55.

17. A vector including the polynucleotide of claim 15.

18. An isolated cell that is transformed with the vector of claim 17.

19. The isolated cell of claim 18, wherein the isolated cell is a T cell, an NK cell, an NKT cell, or a gamma delta ($\gamma\delta$) T cell.

20. A pharmaceutical composition for preventing or treating cancer including the isolated cell of claim 19.

[FIG 1]

| PCR condition | |
|---|---|
| 10X Ex pfu buffer | 5.0 |
| dNTP mix | 2.0 |
| Pfu | 0.5 |
| Primer(upper),10pmol | 2.0 |
| Primer(lower),10pmol | 2.0 |
| Template(50ng/µl) | 1.0 |
| D.W | 37.5 |
| Total | 50 (µl) |

| 95 | 95 | 58 | 72 | 4 |
|---|---|---|---|---|
| 3:00 | 0:30 | 0:30 | 4:00 | ∞ |

34 cycle

[FIG 2]

[FIG 3]

[FIG 4]

## Discovery studio
### MSLN/ScFv34_13-35

[FIG 5]

[FIG 6]

W.T

$R^2=0.994$
$EC_{50}=0.138$

D31W

$R^2=0.994$
$EC_{50}=0.114$

[FIG 7]

D31L

$R^2=0.995$
$EC_{50}=0.087$

S192F

$R^2=0.998$
$EC_{50}=0.078$

[FIG 8]

S192R

Y228R

[FIG 9]

#34(WT)　　　Graph1

R²=0.985
EC₅₀=0.041

D31L　　　Graph2

R²=0.996
EC₅₀=0.018

[FIG 10]

S192R  Graph1

R²=0.998
EC₅₀=0.087

D31L/S192R  Graph2

R²=0.990
EC₅₀=0.010

[FIG 11]

EP 4 613 771 A1

## MSLN-34

EVQLVESGGGLVQPGGSLRLSCAASGFTFS_D_YGMHWVRQAPGKGLEWVSSIYGSGGHTGYADSVKGRFTISRDNSKNTLYLQMNSLRAEDTAVYYCAKQHAYRYSYAFDVW
GQGTLVTVSSGGGGSGGGGSGGGGSDIQMTQSPSSLSASVGDRVTITCRASQSISNWLNWYQQKPGKAPKLLIYATSSLQ_S_GVPSRFSGSGSGTDFTLTISSLQPEDFATYYC
QQSYSFPFTFGQGTKVEIKASTGPGGQHHHHHHGAWSHPQFEK

## MSLN-34

### D31L

EVQLVESGGGLVQPGGSLRLSCAASGFTFS_L_YGMHWVRQAPGKGLEWVSSIYGSGGHTGYADSVKGRFTISRDNSKNTLYLQMNSLRAEDTAVYYCAKQHAYRYSYAFDVW
GQGTLVTVSSGGGGSGGGGSGGGGSDIQMTQSPSSLSASVGDRVTITCRASQSISNWLNWYQQKPGKAPKLLIYATSSLQ_S_GVPSRFSGSGSGTDFTLTISSLQPEDFATYYC
QQSYSFPFTFGQGTKVEIKASTGPGGQHHHHHHGAWSHPQFEK

D31L : GAT → CTT

## MSLN-34

### S192R

EVQLVESGGGLVQPGGSLRLSCAASGFTFS_D_YGMHWVRQAPGKGLEWVSSIYGSGGHTGYADSVKGRFTISRDNSKNTLYLQMNSLRAEDTAVYYCAKQHAYRYSYAFDVW
GQGTLVTVSSGGGGSGGGGSGGGGSDIQMTQSPSSLSASVGDRVTITCRASQSISNWLNWYQQKPGKAPKLLIYATSSLQ_R_GVPSRFSGSGSGTDFTLTISSLQPEDFATYYC
QQSYSFPFTFGQGTKVEIKASTGPGGQHHHHHHGAWSHPQFEK

S192R : TCT → CGT

## MSLN-34

### D31L/S192R

EVQLVESGGGLVQPGGSLRLSCAASGFTFS_L_YGMHWVRQAPGKGLEWVSSIYGSGGHTGYADSVKGRFTISRDNSKNTLYLQMNSLRAEDTAVYYCAKQHAYRYSYAFDVW
GQGTLVTVSSGGGGSGGGGSGGGGSDIQMTQSPSSLSASVGDRVTITCRASQSISNWLNWYQQKPGKAPKLLIYATSSLQ_R_GVPSRFSGSGSGTDFTLTISSLQPEDFATYYC
QQSYSFPFTFGQGTKVEIKASTGPGGQHHHHHHGAWSHPQFEK

D31L : GAT → CTT
S192R : TCT → CGT

[FIG 12]

[FIG 13]

[FIG 14]

[FIG 15]

[FIG 16]

[FIG 17]

NCI-H2052

- Mock
- MSLN34
- MSLN34-D31L
- MSLN34-S192R
- MSLN34-D31L,S192R

OVCAR3

- Mock
- MSLN34
- MSLN34-D31L
- MSLN34-S192R
- MSLN34-D31L,S192R

[FIG 18]

AsPC-1/GFP

Mock
MSLN34
MSLN34-D31L
MSLN34-S192R
MSLN34-D31L,S192R

Batch #1

AsPC-1/GFP

Mock
MSLN34
MSLN34-D31L
MSLN34-S192R
MSLN34-D31L,S192R

Batch #2

[FIG 19]

[FIG 20]

[FIG 21]

[FIG 22]

[FIG 23]

[FIG 24]

[FIG 25]

## INTERNATIONAL SEARCH REPORT

| International application No. |
| --- |
| **PCT/KR2023/017306** |

**A. CLASSIFICATION OF SUBJECT MATTER**

**C07K 16/28**(2006.01)i; **C07K 14/725**(2006.01)i; **A61P 35/00**(2006.01)i; **A61K 39/00**(2006.01)i

According to International Patent Classification (IPC) or to both national classification and IPC

**B. FIELDS SEARCHED**

Minimum documentation searched (classification system followed by classification symbols)

C07K 16/28(2006.01); A61K 35/17(2015.01); A61K 39/00(2006.01); A61K 47/68(2017.01); C07K 14/705(2006.01); C07K 14/725(2006.01); C07K 16/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 메소텔린(mesothelin), 상보성 결정 영역(complementary determining region, CDR), 항체(antibody), 치환(substitution), 암(cancer), 키메릭 항원 수용체(chimeric antigen receptor, CAR)

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2309543 B1 (CELLENGENE INC) 07 October 2021 (2021-10-07)<br>See claims 1-19; and paragraphs [0035]-[0036] and [0127]-[0128]. | 1,8-15,17-20 |
| A | | 2-7,16 |
| A | KR 10-2020-0090598 A (GREENCROSSCELL) 29 July 2020 (2020-07-29)<br>See claims 1-6. | 1-20 |
| A | KR 10-2018-0055824 A (CARSGEN THERAPEUTICS, LTD.) 25 May 2018 (2018-05-25)<br>See claims 1-12. | 1-20 |
| A | US 2016-0311917 A1 (BEATTY, G. et al.) 27 October 2016 (2016-10-27)<br>See entire document. | 1-20 |
| A | WO 2021-130250 A1 (CELLECTIS) 01 July 2021 (2021-07-01)<br>See entire document. | 1-20 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **02 February 2024** | **05 February 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/017306**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

| International application No. |
|---|
| **PCT/KR2023/017306** |

| Patent document cited in search report | | | Publication date (day/month/year) | Patent family member(s) | | | Publication date (day/month/year) |
|---|---|---|---|---|---|---|---|
| KR | 10-2309543 | B1 | 07 October 2021 | AU | 2021-245153 | A1 | 24 February 2022 |
| | | | | AU | 2021-245153 | B2 | 30 June 2022 |
| | | | | CA | 3133678 | A1 | 14 January 2022 |
| | | | | CA | 3133678 | C | 11 April 2023 |
| | | | | CN | 114364702 | A | 15 April 2022 |
| | | | | CN | 114364702 | B | 16 September 2022 |
| | | | | EP | 4194467 | A1 | 14 June 2023 |
| | | | | JP | 2022-543333 | A | 12 October 2022 |
| | | | | JP | 7138989 | B1 | 20 September 2022 |
| | | | | US | 11795215 | B2 | 24 October 2023 |
| | | | | US | 2023-0151082 | A1 | 18 May 2023 |
| | | | | WO | 2022-030730 | A1 | 10 February 2022 |
| KR | 10-2020-0090598 | A | 29 July 2020 | CN | 113784986 | A | 10 December 2021 |
| | | | | EP | 3916019 | A1 | 01 December 2021 |
| | | | | JP | 2022-518548 | A | 15 March 2022 |
| | | | | JP | 7182012 | B2 | 01 December 2022 |
| | | | | KR | 10-2070016 | B1 | 29 January 2020 |
| | | | | US | 2021-0347870 | A1 | 11 November 2021 |
| | | | | WO | 2020-153605 | A1 | 30 July 2020 |
| KR | 10-2018-0055824 | A | 25 May 2018 | AU | 2016-312015 | A1 | 12 April 2018 |
| | | | | BR | 112018003339 | A2 | 21 November 2018 |
| | | | | CA | 2996060 | A1 | 02 March 2017 |
| | | | | CL | 2018000457 | A1 | 24 August 2018 |
| | | | | CN | 106467573 | A | 01 March 2017 |
| | | | | CN | 106467573 | B | 25 May 2021 |
| | | | | EP | 3339326 | A1 | 27 June 2018 |
| | | | | HK | 1251948 | A1 | 03 May 2019 |
| | | | | IL | 257563 | A | 30 April 2018 |
| | | | | JP | 2018-529327 | A | 11 October 2018 |
| | | | | JP | 6994456 | B2 | 04 February 2022 |
| | | | | NZ | 740855 | A | 29 April 2022 |
| | | | | RU | 2018107802 | A | 23 September 2019 |
| | | | | RU | 2748281 | C2 | 21 May 2021 |
| | | | | SG | 11201801351 | A | 28 March 2018 |
| | | | | US | 10793641 | B2 | 06 October 2020 |
| | | | | US | 2018-0244796 | A1 | 30 August 2018 |
| | | | | WO | 2017-032293 | A1 | 02 March 2017 |
| US | 2016-0311917 | A1 | 27 October 2016 | AR | 098848 | A1 | 15 June 2016 |
| | | | | AU | 2014-366047 | A1 | 16 June 2016 |
| | | | | AU | 2014-366047 | B2 | 25 March 2021 |
| | | | | AU | 2021-203547 | A1 | 01 July 2021 |
| | | | | AU | 2021-203547 | B2 | 24 November 2022 |
| | | | | AU | 2023-200884 | A1 | 11 May 2023 |
| | | | | BR | 112016013187 | A2 | 26 September 2017 |
| | | | | CA | 2931684 | A1 | 25 June 2015 |
| | | | | CN | 106459989 | A | 22 February 2017 |
| | | | | CN | 106459989 | B | 17 February 2023 |
| | | | | CN | 116478927 | A | 25 July 2023 |
| | | | | EP | 3083964 | A1 | 26 October 2016 |
| | | | | EP | 3083964 | B1 | 26 January 2022 |

Form PCT/ISA/210 (patent family annex) (July 2022)

## INTERNATIONAL SEARCH REPORT
### Information on patent family members

International application No.

**PCT/KR2023/017306**

| Patent document cited in search report | Publication date (day/month/year) | Patent family member(s) | | Publication date (day/month/year) |
|---|---|---|---|---|
| | | EP | 4026909 A1 | 13 July 2022 |
| | | ES | 2918501 T3 | 18 July 2022 |
| | | HK | 1224332 A1 | 18 August 2017 |
| | | IL | 245764 A | 31 July 2016 |
| | | JP | 2017-500869 A | 12 January 2017 |
| | | JP | 2021-035948 A | 04 March 2021 |
| | | JP | 2022-046476 A | 23 March 2022 |
| | | JP | 2023-159154 A | 31 October 2023 |
| | | JP | 6779785 B2 | 04 November 2020 |
| | | KR | 10-2016-0098259 A | 18 August 2016 |
| | | MX | 2016008076 A | 12 August 2016 |
| | | RU | 2016129125 A | 23 January 2018 |
| | | RU | 2714902 C2 | 20 February 2020 |
| | | SG | 11201604815 A | 28 July 2016 |
| | | TW | 201529850 A | 01 August 2015 |
| | | TW | I672379 B | 21 September 2019 |
| | | US | 10640569 B2 | 05 May 2020 |
| | | US | 2020-0407460 A1 | 31 December 2020 |
| | | WO | 2015-090230 A1 | 25 June 2015 |
| WO 2021-130250 A1 | 01 July 2021 | AU | 2020-415318 A1 | 14 July 2022 |
| | | CA | 3166356 A1 | 01 July 2021 |
| | | CN | 115175928 A | 11 October 2022 |
| | | EP | 4081537 A1 | 02 November 2022 |
| | | IL | 294118 A | 01 August 2022 |
| | | JP | 2023-507525 A | 22 February 2023 |
| | | KR | 10-2022-0118532 A | 25 August 2022 |
| | | MX | 2022007833 A | 23 September 2022 |
| | | US | 2023-0068949 A1 | 02 March 2023 |

Form PCT/ISA/210 (patent family annex) (July 2022)

**EP 4 613 771 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Cancer Discov.*, February 2016, vol. 6 (2), 133-46
- *J Reprod Immunol.*, 2020, vol. 139, 103115
- *Gynecol Oncol.*, 2007, vol. 105 (3), 563-570
- *Eur J Haematol.*, 2007, vol. 79 (4), 281-286